# EUROPEAN PATENT APPLICATION

(11) **EP 4 767 966 A1**
(43) Date of publication of application: **01.07.2026**
(21) Application number: 25218535.0
(22) Date of filing: 26.11.2025
(51) Int. Cl.: A61B 18/14, A61B 18/12

(54) **INTEGRATED FOCAL ABLATION CATHETER AND EXPANDABLE MAPPING AND ABLATION CATHETER**

(30) Priority: 31.12.2024 US 202419006529
(71) Applicant: Biosense Webster (Israel) Ltd., 2066717 Yokneam (IL)
(72) Inventor: VAN NIEKERK, Pieter Emmelius, Irvine, 92618 (US); BASU, Shubhayu, Irvine, 92618 (US); HIGHSMITH, Debby, Irvine, 92618 (US); SUAREZ, Paul, Irvine, 92618 (US); HENRIQUEZ, Jamie Lynn, Irvine, 92618 (US); BAR-TAL, Meir, 2066717 Yokneam (IL); BERGER, Abraham, 2066717 Yokneam (IL); BERGER, Omer, 2066717 Yokneam (IL)
(74) Representative: Carpmaels & Ransford LLP

(57) **Abstract**

Disclosed is a medical probe comprising an elongated shaft extending along a longitudinal axis and a distal electrode disposed at a distal end of the elongated shaft, the distal electrode configured to deliver ablative energy to tissue. The medical probe includes an outer sleeve disposed at least partially around the elongated shaft and a plurality of planar members attached to the outer sleeve and extending radially outward from the longitudinal axis. Each planar member of the plurality of planar members comprises a framework coupled to an outer surface member and an inner surface member opposite the outer surface member. Each of the inner and outer surface members includes a plurality of electrodes diametrically disposed on the inner surface member and outer surface member and the outer sleeve is configured to move axially along the elongated shaft to move the plurality of planar members axially with respect to the distal electrode.

## Description

### FIELD

The present invention relates generally to medical devices, and in particular to catheters configured to ablate tissue by pulse field ablation.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Arrhythmia is treated surgically or by catheter ablation to cease or modify the propagation of unwanted electrical signals from one portion of the heart to another.

Many contemporaneous ablation approaches utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that can reduce some thermal risks associated with RF ablation but may present tissue damage due to the very low temperature nature of such devices. Maneuvering cryoablation devices and selectively applying cryoablation is generally more challenging compared to RF ablation; therefore, cryoablation is not viable in certain anatomical geometries which may be reached by RF ablation devices.

Pulse field ablation (PFA) is a comparatively new, non-thermal method of ablating cardiac tissue by causing irreversible electroporation (IRE) in cells of target tissue. To achieve IRE, short pulses of high voltage electrical signals are delivered to tissue and the electrical signals generate an unrecoverable permeabilization of cell membranes. Examples of systems and devices configured for IRE ablation are disclosed in U.S. Patent Pub. No. 2021/0169550A1, 2021/0169567A1, 2021/0169568A1, 2021/0196372A1, 2021/0177503A1, and 2021/0186604A1, and U.S. Pat. No. 11,540,877 each of which are incorporated herein by reference in their entireties and attached in the Appendix hereto.

Regions of cardiac tissue can be mapped by a catheter to identify the abnormal electrical signals. Some catheter ablation procedures especially those with persistent atrial fibrillation may be performed using electrophysiology (EP) mapping to target areas of aberrant electrical signals. Such EP mapping may include the use of diagnostic electrodes configured to monitor electrical signals within the cardiovascular system to pinpoint the location of aberrant conductive tissue sites that are responsible for the arrhythmia. Examples of an EP mapping system are described in U.S. Patent No. 5,738,096, incorporated herein in its entirety by reference and attached in the Appendix hereto. Examples of EP mapping catheters are described in U.S. Patent No. 9,907,480, U.S. Patent Pub. No. 2018/0036078, and U.S. Patent Pub. No. 2018/0056038, each of which are incorporated herein by reference in their entireties.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, Calif.

In many catheter ablation procedures, physicians typically first map the tissue using a mapping catheter having a large surface area (such as a balloon catheter, a basket catheter, or other catheter having multiple mapping electrodes) and then insert either a focal ablation catheter having a single electrode at a distal end to ablate small areas of tissue or an expandable ablation catheter having multiple electrodes to ablate large areas. This process generally requires the physician to remove the mapping catheter before inserting the focal ablation catheter to ablate the tissue. As will be appreciated, inserting and removing multiple catheters elongates the time required to ablate the tissue and can cause additional stress on the patient's cardiovascular system. These and other problems are addressed by the technology disclosed herein.

### SUMMARY

The disclosed technology includes a medical probe comprising an elongated shaft extending along a longitudinal axis and a distal electrode disposed at a distal end of the elongated shaft. The distal electrode can be configured to deliver ablative energy to tissue. The medical probe further comprises an outer sleeve disposed at least partially around the elongated shaft and a plurality of planar members attached to the outer sleeve and extending radially outward from the longitudinal axis. Each planar member of the plurality of planar members comprises a framework coupled to an outer surface member and an inner surface member opposite the outer surface member. Each of the inner and outer surface members includes a plurality of electrodes diametrically disposed on the inner surface member and outer surface member and the outer sleeve is configured to move axially along the elongated shaft to move the plurality of planar members axially with respect to the distal electrode.

The disclosed technology further includes a method comprising inserting a medical probe into a lumen of a body. The medical probe comprises an elongated shaft extending along a longitudinal axis and a distal electrode disposed at a distal end of the elongated shaft. The distal electrode can be configured to deliver ablative energy to tissue. The medical probe further comprises an outer sleeve disposed at least partially around the elongated shaft and a plurality of planar members attached to the outer sleeve and extending radially outward from the longitudinal axis. Each planar member of the plurality of planar members comprises a plurality of electrodes and the outer sleeve can be configured to move axially along the elongated shaft to move the plurality of planar members axially with respect to the distal electrode. The method can further include sliding the outer sleeve along the elongated shaft to cause the plurality of planar members to extend beyond a distal end of the distal electrode and receiving one or more electrophysiological signals from at least one electrode of the plurality of electrodes disposed on the plurality of planar members.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements. The FIGs. depict one or more implementations of the inventive devices, by way of example only, not by way of limitation.
FIG. 1 is an illustration of an example catheter-based electrophysiology mapping and ablation system, according to an example of the disclosed technology.
FIG. 2A is an illustration of a mapping and ablation catheter having both a focal end effector portion and an expandable end effector portion with the focal end effector portion in an extended configuration, according to an example of the disclosed technology.
FIG. 2B is an illustration of a mapping and ablation catheter having both a focal end effector portion and an expandable end effector portion with the focal end effector portion in a retracted configuration, according to an example of the disclosed technology.
FIG. 3A is an illustration of the focal end effector portion in an extended configuration and an expandable end effector portion in an expanded configuration, according to an example of the disclosed technology.
FIG. 3B is an illustration of the focal end effector portion in a retracted configuration and an expandable end effector portion in an expanded configuration, according to an example of the disclosed technology.
FIG. 3C is an illustration of another example of the catheter with the focal end effector portion in an extended configuration and the expandable end effector portion in an expanded configuration, according to an example of the disclosed technology.
FIG. 3D is an illustration of the example catheter shown in FIG. 3D with the focal end effector portion in a retracted configuration and an expandable end effector portion in an expanded configuration, according to an example of the disclosed technology.
FIG. 4 is a detailed view of the focal end effector portion, according to an example of the disclosed technology.
FIG. 5A is a detail view of the expandable end effector portion, according to an example of the disclosed technology.
FIG. 5B is a front view of a planar member of the expandable end effector portion, according to an example of the disclosed technology.
FIG. 5C is a magnified view of a portion of the planar member of the expandable end effector portion shown in FIG. 5B, according to an example of the disclosed technology.
FIG. 6A is an illustration of a first example electrode configuration for the example planar member of the expandable end effector portion including elongated serpentine ablation electrodes and diagnostic electrodes positioned alongside the ablation electrodes.
FIG. 6B is an illustration of a second example electrode configuration for the example planar member of the expandable end effector portion including elongated ablation electrodes which are respectively segmented into small parallel stripes and diagnostic electrodes positioned alongside the ablation electrodes.
FIG. 6C is an illustration of a third example electrode configuration for the example planar member of the expandable end effector portion including rectangular elongated ablation electrodes without diagnostic electrodes.
FIG. 6D is an illustration of a fourth example electrode configuration for the example planar member of the expandable end effector portion including rectangular elongated ablation electrodes interrupted by diagnostic electrodes.
FIG. 6E is an illustration of an example electrode configuration for the example end effector including elongated serpentine ablation electrodes.
FIG. 6F is an illustration of an example electrode configuration for the example end effector including rectangular elongated ablation electrodes.
FIG. 6G is an illustration of an example electrode configuration for the example end effector including large area ablation electrodes.
FIG. 7 is an illustration of an example electrode configuration on one side the example planar member of the expandable end effector portion including elongated ablation electrodes in two ablation electrode regions, diagnostic electrodes positioned in the two ablation electrode regions, tissue contact electrodes positioned between the two ablation electrode regions, and a reference electrode positioned in a proximal region of the illustrated side of the end effector.
FIGs. 8A-8D are illustrations of alternative configurations of insulating material in a cross-sectional view of the planar member in which FIG. 8A is configured with openings in the end effector as indicated in FIG. 5B; FIG. 8B includes a polymeric body region extending a width of the end effector; FIG. 8C includes a high dielectric layer between each side and extending a width of the end effector; and FIG. 8D includes high dielectric tiles between each side and configured to overlap to collapse the end effector into a delivery sheath.
FIG. 8E is an illustration of an example electrode configuration for the example end effector in which ablation electrodes wrap around a spine or insulative material of the planar member.
FIG. 8F is an illustration of an example electrode configuration for the example end effector in which ablation electrodes partially wrap around a spine or insulative material of the planar member.
FIG. 9A is an illustration of the end effector in which the ablation electrodes are configured to provide between 600 V and 2,600 V pulses between ablation electrode regions.
FIG. 9B is an illustration of the end effector applying the treatment illustrated in FIG. 9B to a potato model.

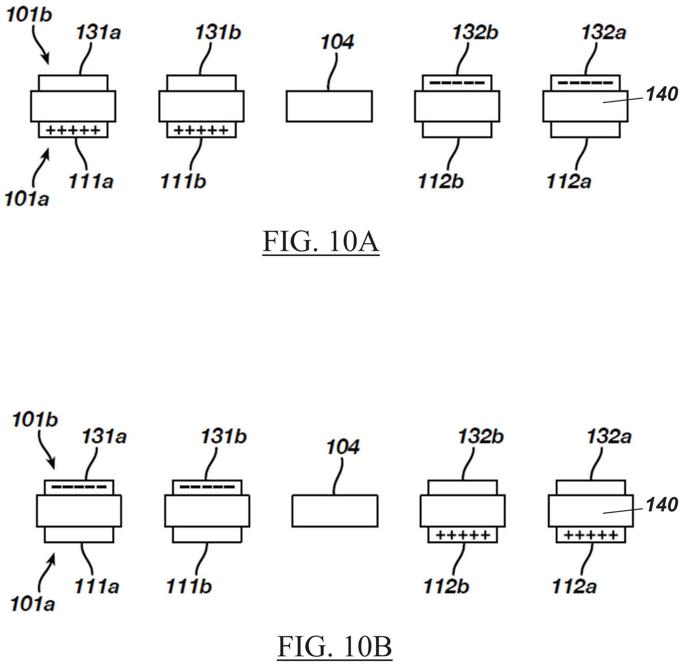

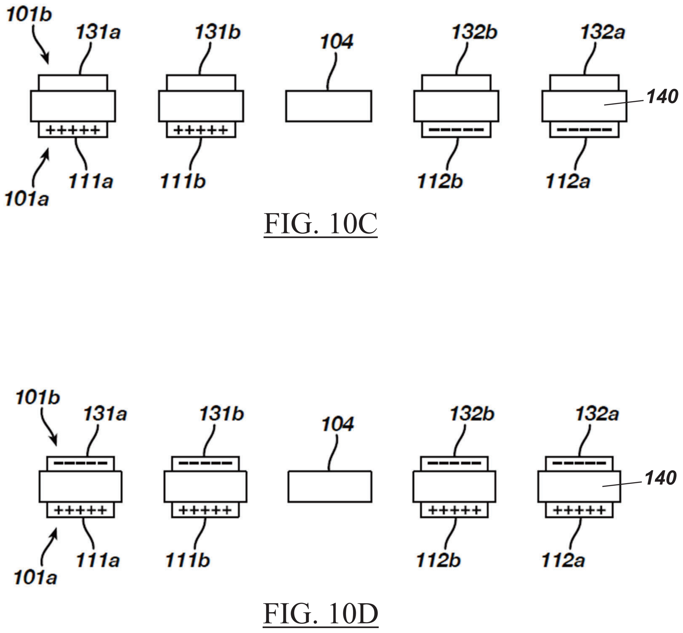

FIGs. 10A-10D illustrate various examples of voltage being applied across the electrodes of the planar member, according to examples of the disclosed technology. FIGs. 10A and 10B illustrate a voltage applied across electrode pairs in a cross-body configuration in which, for each pair, one ablation electrode is in contact with tissue and the other electrode is on an opposite side of the end effector body and across a central axis of the end effector body from the first electrode region; FIG. 10C illustrates a voltage applied across ablation electrode pairs in which both ablation electrodes in the pair are in contact with tissue and positioned across the central axis from each other; and FIG. 10D illustrates a voltage applied across ablation electrodes in contact with tissue to ablation electrodes on an opposite side of the end effector and not in contact with tissue.
FIGs. 10E and 10F illustrate a voltage applied across electrode pairs of an example planar member in which electrodes of the pair are positioned across the central axis from each other.
FIG. 10G illustrates a voltage applied across electrodes on the same side of the central axis.
FIG. 11 is an illustration of an example electrode configuration on one side of the planar member of the expandable end effector portion including elongated ablation electrodes in four ablation electrode regions, diagnostic electrodes positioned in at least two of the ablation electrode regions, tissue contact electrodes positioned along the longitudinal axis between ablation electrode regions, and a reference electrode positioned in a proximal region of the illustrated side of the end effector.
FIGs. 12A and 12B illustrate additional examples of performing ablation using electrodes on the various planar members of the expandable end effector portion, according to an example of the disclosed technology.
FIG. 13A is a flow chart of an example method of using a mapping and ablation catheter having both a focal end effector portion and an expandable end effector portion, according to an example of the disclosed technology.
FIG. 13B is a flow chart of another example method of using a mapping and ablation catheter having both a focal end effector portion and an expandable end effector portion, according to an example of the disclosed technology.

### DETAILED DESCRIPTION

The disclosed technology includes a catheter end effector that includes both a focal end effector portion and an expandable end effector portion. The expandable end effector portion can be configured to move axially along the longitudinal axis with respect to the focal end effector portion. That is, the expandable end effector portion can slide over the focal end effector portion such that either the focal end effector portion or the expandable end effector portion can be used alone without the other. In this way, the focal end effector portion can be used to map or ablate small areas of tissue while the expandable end effector portion can be used to map or ablate large areas of tissue as necessary. Thus, the disclosed technology can simplify mapping and ablation procedures because a single catheter can be used for both large and small areas of tissue instead of requiring multiple catheters for the procedure.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

In addition, as used herein, the terms "patient," "host," "user," and "subject" refer to any human or animal subject and are not intended to limit the systems or methods to human use, although use of the subject invention in a human patient represents a preferred embodiment.

The term "proximal" indicates a location closer to the operator whereas "distal" indicates a location further away to the operator or physician.

As discussed herein, "operator" and "user" can include a doctor, surgeon, technician, scientist, or any other individual or delivery instrumentation associated with delivery of a multi-electrode catheter for treatments disclosed herein.

As discussed herein, the terms "bipolar" and "unipolar" when used to refer to IRE ablation schemes describe ablation schemes which differ with respect to electrical current path and electric field distribution. "Bipolar" refers to an IRE ablation scheme utilizing a current path between two electrodes that are both positioned near an ablation site or inside the organ to be treated; current density and electric flux density is typically, but not necessarily, approximately equal at each of the two electrodes. "Unipolar" refers to an IRE ablation scheme utilizing a current path between two electrodes where one electrode having a high current density and high electric flux density is positioned at a treatment site, and a second electrode having comparatively lower current density and lower electric flux density is positioned remotely from the treatment site such as on a patch on the patient's skin usually outside the body.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Each phase of the biphasic and monophasic pulse preferably has a square shape having an essentially constant voltage amplitude during a majority of the phase duration. Phases of the biphasic pulse may be separated in time by an interphase delay.

To the extent that any materials incorporated by reference herein contain similar terms but differ in definition or description, it will be appreciated that the definitions and/or descriptions provided herein are to be used in understanding the technology disclosed herein.

Alternative apparatus and system features and alternative method steps are presented in example embodiments herein. Each given example embodiment presented herein can be modified to include a feature and/or method step presented with a different example embodiment herein where such feature and/or step is compatible with the given example as understood by a person skilled in the pertinent art as well as where explicitly stated herein. Such modifications and variations are intended to be included within the scope of the claims.

A catheter is presented having an end effector with a focal end effector portion and an expandable end effector portion with elongated ablation electrodes configured to provide electrical signals to achieve IRE in target tissue. The expandable end effector portion can have a plurality of planar members configured to flex away from the longitudinal axis when in an expanded configuration and when contacting tissue. In some embodiments, the catheter is compatible with an 8.5 French sheath. In some embodiments, the planar members each include a highly insulative layer separating electrodes on either side of the end effector. In order to facilitate collapse of highly rigid insulators, in some embodiments, the highly insulative layer includes tiles of insulating material that overlap when the end effector is retracted into the sheath.

As discussed herein for illustrative purposes, the planar members of the expandable end effector include four elongated electrode segments on each side of the planar member (total of eight elongated electrode segments). The elongated electrode segments extend from approximate the distal end of the planar member to about half, or more than half of the length of the planar member as measured from its distal end to a proximal end of the planar member. The planar member can include two or more electrode regions per side of the planar member (four or more electrode regions total) such that each electrode region includes two of the elongated electrode segments connected within the end effector to function as a singular electrode. Alternatively, each of the electrode segments can be bisected approximately in half resulting in twice as many elongated electrode segments each above half as long as the previously described elongated electrode segments. This embodiment can include four or more electrode regions per side of the end effector, eight or more electrode regions total. As will be appreciated, the number of electrodes (including electrode regions and electrode segments) can be greater or fewer than those offered herein for illustrative purposes. That is, although some examples of the planar members are described herein and illustrated as having a specific number of electrodes, one of skill in the art will appreciate that the disclosed technology can include greater or fewer numbers of electrodes without departing from the scope of this disclosure.

An electrical signal including pulses to achieve IRE can be applied between pairs of electrodes to achieve PFA. The ablation electrodes can be paired in a variety of ways and pulses can be applied between different pairs in the same PFA treatment. In some embodiments, the end effector may provide PFA to create a lesion with a depth of approximately 7.5 mm in a potato model. Bipolar electrical signals can be applied between the elongated ablation electrodes in various pair combinations to achieve PFA.

Each ablation electrode can be designed with shape based on trade-off between total edge area, total contact area, and open space for non-ablation electrodes. Applicants recognize that a larger ratio of surface area to perimeter may effectively deliver the ablation energy while reducing the chance of electrical arcing. Applicants recognize that it is desirable to avoid arcing. Conversely, serpentine electrodes increase mechanical flexibility of the paddle. Applicants recognize that flexibility of the electrode may result in improved tissue contact and catheter longevity, but the geometry of the serpentine electrode has a lower area to perimeter ratio compared to a solid electrode.

A challenge with existing ablation catheters, and PFA catheters in particular, is integrating diagnostic electrodes into the end effector assembly so that tissue can be mapped and ablated by the same end effector. In some embodiments, the end effector presented herein may include diagnostic electrodes on the planar members of the expandable end effector that are configured to receive electrical signals from tissue to map tissue in addition to the ablation electrodes. Configured as such, the expandable end effector can be configured for both mapping and ablation. Alternatively, or in addition, the focal end effector can be configured to receive electrical signals from tissue to map tissue in addition to the ablation electrode. In this way, the focal end effector can similarly be configured for both mapping and ablation.

Another challenge with existing ablation catheters is that they are generally designed for ablating only large areas (e.g., balloon, basket, planar, and other expandable catheters) of tissue or only small areas of tissue (e.g., focal ablation catheter). The disclosed technology includes a catheter having both a focal end effector portion and an expandable end effector portion such that the disclosed catheter is capable of ablating large areas of tissue or small areas of tissue.

In some embodiments, the focal end effector and/or the expandable end effector includes pairs of tissue contact electrodes to determine which portions of the end effector are in contact with tissue.

In some embodiments, the focal end effector and/or the expandable end effector includes reference electrodes configured to measure blood voltage. The ablation electrodes, diagnostic electrodes, or tissue contact electrodes may be used as a reference electrode when not in contact with tissue. The focal end effector and/or the expandable end effector may additionally or alternatively include one or more dedicated reference electrodes positioned on a portion of the end effector or shaft near the treatment or diagnostic electrodes in a location not likely to be in contact with tissue during a procedure, such as on a proximal portion of the focal end effector and/or the expandable end effector.

Aspects of the catheter, end effector, and related methods and systems are described in relation to the figures as follows.

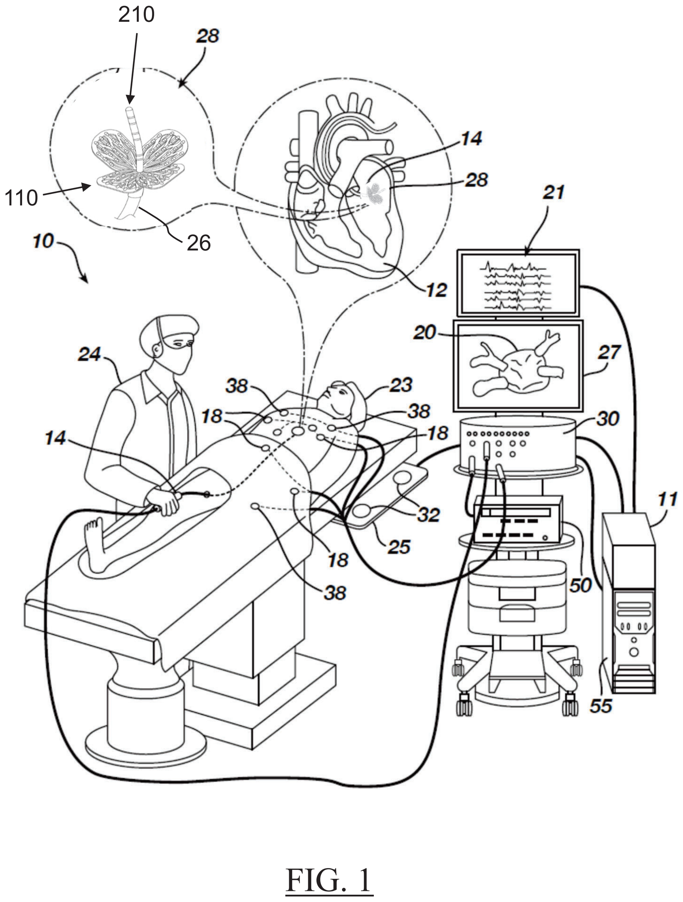

FIG. 1 is an illustration showing an example catheter-based electrophysiology mapping and ablation system 10. The system 10 may include multiple catheters, which are percutaneously inserted by a physician 24 through the patient's vascular system into a chamber or vascular structure of a heart 12. Typically, a delivery sheath catheter (e.g. 8.5 French) is inserted into the left or right atrium near a desired location in the heart 12. Thereafter, catheter(s) can be inserted into the delivery sheath catheter so as to arrive at the desired location. Such catheter(s) may include catheters dedicated for sensing Intracardiac Electrogram (IEGM) signals, catheters dedicated for ablating, and/or catheters dedicated for both sensing and ablating.

An example catheter 14 (sometimes referred to herein as medical probe 14) including an end effector 28 that is configured for providing PFA is illustrated herein. The end effector 28 includes ablation electrodes configured to provide PFA and optionally diagnosis electrodes, tissue contact electrodes, and at least one reference electrode. In some embodiments, the end effector 28 includes diagnostic electrodes configured for mapping aberrant electrical signals in the heart to detect atrial fibrillation and is thus the end effector 28 may be configured for both sensing and mapping. In such examples, the diagnostic electrodes are on the same side of the end effector and in close proximity to the ablation electrodes. The physician 24 brings the end effector 28 into contact with the heart wall for sensing a target site in the heart 12 and providing ablation treatment to the target site without the need to reposition the end effector between a mapping step and an ablation step. Electrode arrangements are shown in greater detail in subsequent figures. Electrodes may be used for more than one purpose as understood by a person skilled in the art informed by the examples presented herein.

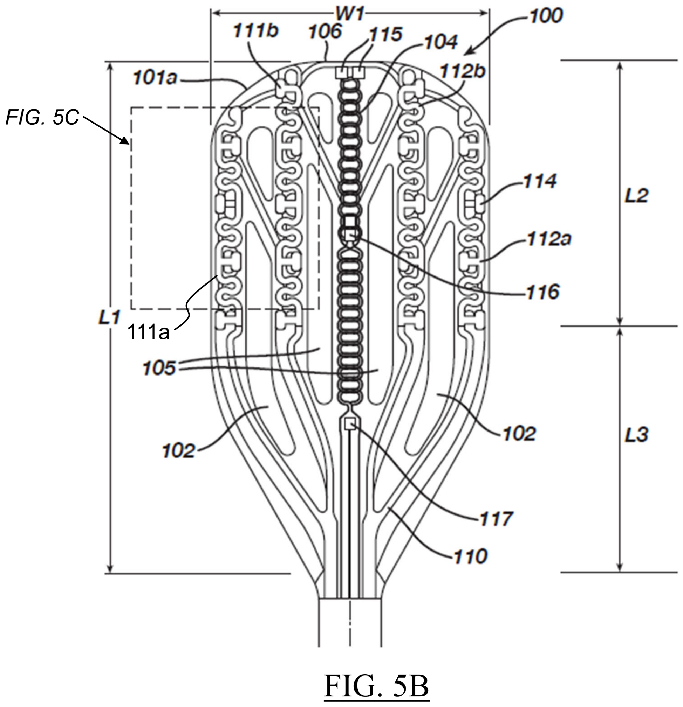

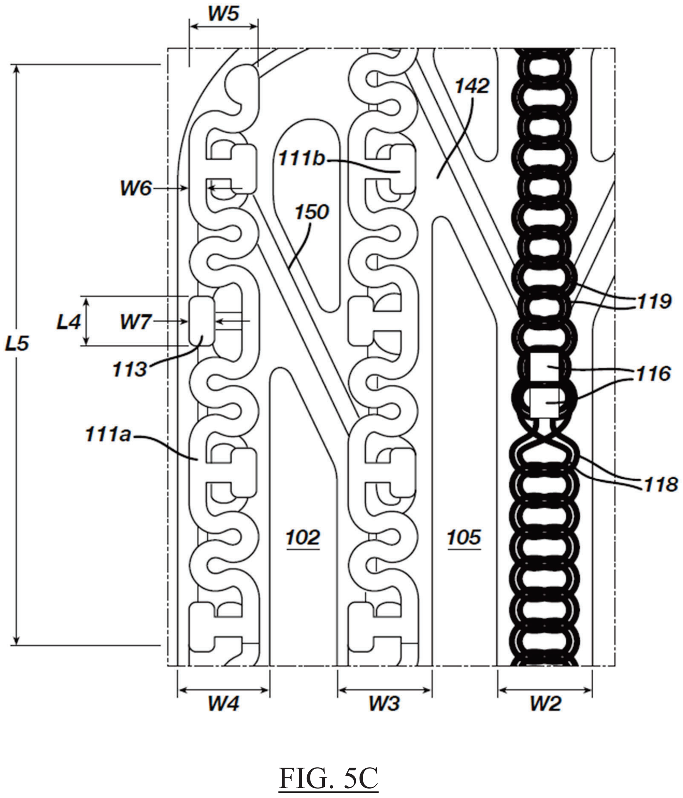

A magnetic based position sensor 29 may be operated together with a location pad 25 including a plurality of magnetic coils 32 configured to generate magnetic fields in a predefined working volume. Real time position of the end effector 28 of the catheter 14 may be tracked based on magnetic fields generated with a location pad 25 and sensed by a magnetic based position sensor 29 disposed in the catheter shaft 90. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091 incorporated in their entireties by reference herein. The end effector 28 may further include one or more inductive coils configured to provide electrical signals to the magnetic based position sensing system to determine location and/or orientation of the end effector. For instance, the end effector 28 may include inductive loops or coils similar to as illustrated in FIGs. 5B and 5C of U.S. Patent Publication No. 2024/0215894 incorporated by reference in its entirety herein and attached in the Appendix hereto. In some embodiments, one or more inductive coils in the end effector may be used together with position sensor 29 to determine location and/or orientation of the end effector.

The system 10 includes one or more electrode patches 38 positioned for skin contact on the patient 23 to establish location reference for location pad 25 as well as impedance-based tracking of electrode(s) 26. For impedance-based tracking, electrical current is directed toward electrode(s) 26 and sensed at electrode skin patches 38 so that the location of each electrode can be triangulated via the electrode patches 38. The illustration shows an impedance-based tracking electrode 26 affixed to the catheter shaft. Additionally, or alternatively, the end effector 28 may include one or more impedance-based tracking of electrode(s). Details of the impedance-based location tracking technology are described in US Patent Nos. 7,536,218; 7,756,576; 7,848,787; 7,869,865; and 8,456,182 incorporated by reference herein in their entireties.

A recorder 11 displays electrograms 21 captured with body surface ECG electrodes 18. In embodiments in which the end effector 28 includes diagnostic electrodes, the recorder 11 may further display intracardiac electrograms (IEGM) derived from electrical signals from diagnostic electrodes on the end effector 28. The recorder 11 may include pacing capability for pacing the heart rhythm and/or may be electrically connected to a standalone pacer.

The system 10 includes an ablation energy generator 50 that is adapted to conduct ablative energy to ablation electrodes on the end effector 28. Energy produced by the ablation energy generator 50 includes pulses to induce IRE to ablate via PFA and may also be configured to provide radiofrequency (RF) energy to warm tissue to facilitate PFA with no significant thermal ablation or heat tissue to thermally ablate in addition to PFA. The ablation energy generator 50 is preferably configured to provide biphasic bipolar pulses to induce IRE while keeping tissue temperature below thermal ablation temperatures. Additionally, or alternatively the ablation generator 50 is configured to provide monophasic IRE pulses, unipolar IRE pulses, thermal ablation electrical signals, or combinations thereof. For instance, the ablation energy generator 50 can be configured to provide pulses similar to as described in in U.S. Patent Pub. No. 2021/0169550A1, 2021/0177503A1, 2021/0186604A1, and 2023/0009191A1 and U.S. Patent No. 11,540,877B2, each of which are incorporated herein by reference in their entireties and attached in the Appendix hereto. U.S. Patent No. 11,540,877B2 corresponds to U.S. Patent Pub. No. 2021/0161592A1, which is incorporated here by reference in its entirety.

For instance, as described in U.S. Patent No. 11,540,877B2, the generator 50 can be configured to apply bipolar pulses having an amplitude sufficient to cause IRE in the tissue contacted by the electrodes and also RF energy having power sufficient to thermally ablate the tissue contacted by the electrodes. In some embodiments, the sequence of bipolar pulses includes pulses having an amplitude of at least 200 V, and a duration of each of the bipolar pulses is less than 20 µs. Additionally, or alternatively, the RF signal has a frequency between 350 and 500 kHz and an amplitude between 10 and 200 V. The end effector may further include temperature sensors and the electrical signal generator can be configured to apply the signals responsively to a temperature measured by the temperature sensors. In some embodiments, the IRE signal may have parameters as indicated in Table 1 of U.S. Patent No. 11,540,877B2. Note that the "bipolar pulse" as described in U.S. Patent No. 11,540,877B2 is referred as a "biphasic pulse" herein which relates to the shape of an electrical signal; whereas a "bipolar pulse" as described herein relates to the arrangement of electrodes receiving the pulse as defined herein.

A patient interface unit (PIU) 30 is an interface configured to establish electrical communication between catheters, electrophysiological equipment, power supply, and a workstation 55 for controlling operation of system 10. Electrophysiological equipment of the system 10 may include for example, multiple catheters, the location pad 25, body surface ECG electrodes 18, electrode patches 38, the ablation energy generator 50, and the recorder 11. Optionally and preferably, the PIU 30 includes processing capability for implementing real-time computations of location of the catheters and for performing ECG calculations. The PIU 30 can control the generator 50 to provide electrical energy to the ablation electrodes of the end effector 28 according to the ablation protocols described above and in the above incorporated references. The PIU 30, workstation 55, and the generator 50 can collectively be considered an ablation system console having one or more output ports configured to provide ablation energy to the ablation electrodes, one or more processors, and non-transitory computer-readable medium in communication with the processor to cause the ablation system console to provide ablation energy as described above and in the examples herein below.

In some embodiments, the system 10 further includes an irrigation system configured to irrigate during IRE. For example, the end effector 28 can include one or more irrigation ports and the irrigation system can be configured to deliver irrigation to the one or more irrigation ports. In some embodiments, the PIU 30 is configured to control the irrigation system to provide irrigation to the end effector 28 similar to as described in U.S. Patent Pub. No. 2021/0196372A1 incorporated by reference in its entirety herein and attached in the Appendix hereto. The irrigation fluid may exit the distal end of the catheter 14 through a port at the distal end of the shaft and/or through pores in the body of the end effector 28.

The workstation 55 includes memory, processor unit with memory or storage with appropriate operating software loaded therein, and user interface capability. The workstation 55 can be configured to provide multiple functions, optionally including (1) modeling the endocardial anatomy in three-dimensions (3D) and rendering the model or an anatomical map 20 for display on a display device 27; (2) displaying on the display device 27 activation sequences (or other data) compiled from recorded electrograms 21 in representative visual indicia or imagery superimposed on the rendered anatomical map 20; (3) displaying real-time location and orientation of one or more catheters within the heart chamber; and (4) displaying on the display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

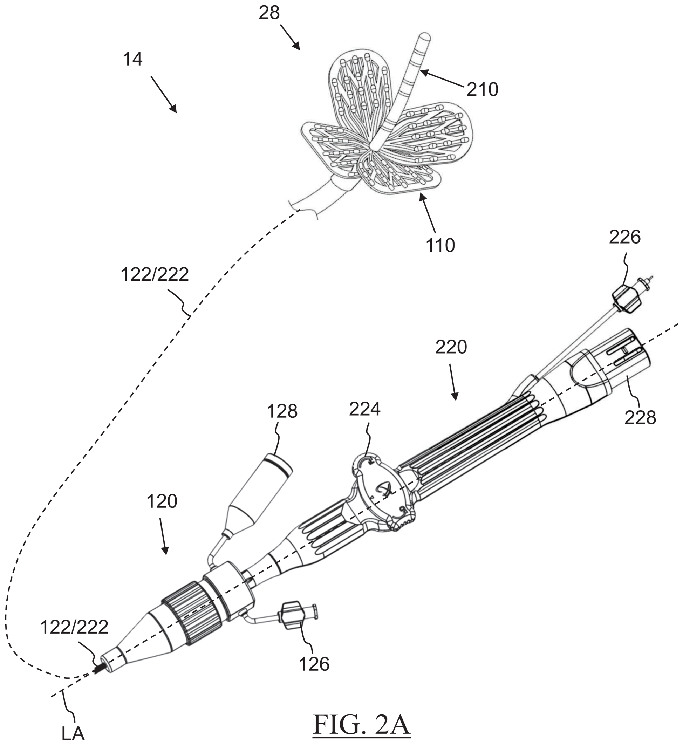

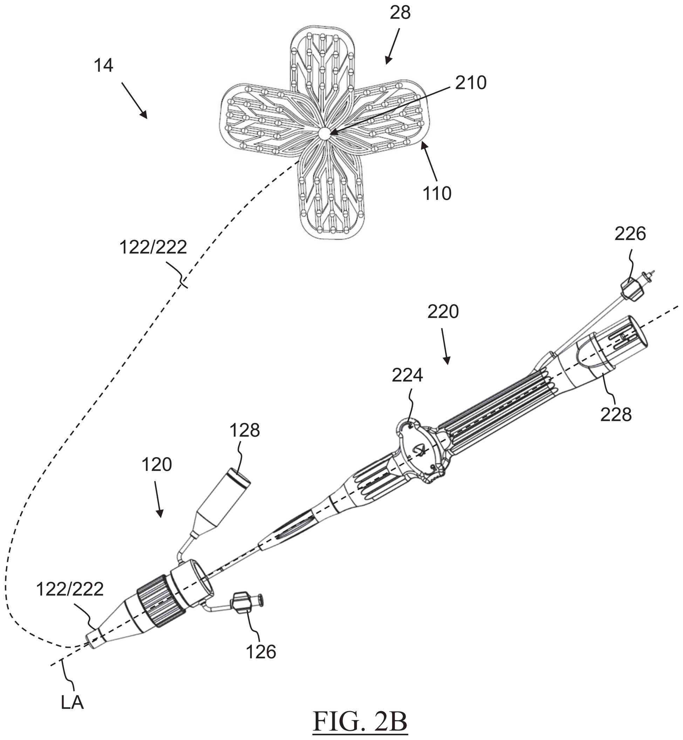

FIGs. 2A and 2B show the catheter 14 in greater detail. The catheter 14 (sometimes referred to herein as a medical probe 14) can be configured to be inserted into a heart 12 of a patient 23 for mapping and ablating tissue in the heart 12. As shown, the catheter 14 can include an end effector 28, a first handle 120, and a second handle 220. The end effector 28 can be attached to the first handle 120 by an outer sleeve 122 and the second handle 220 by an inner catheter shaft 222 (e.g., an elongated shaft). The sleeve 122 can be disposed around the inner catheter shaft 222 such that the sleeve 122 can slide along the outside of the inner catheter shaft 222.

The end effector 28 can further include a focal end effector portion 210 and an expandable end effector portion 110. The focal end effector portion 210 is attached to a distal end of the inner catheter shaft 222 while the expandable end effector portion 110 is attached to a distal end of the sleeve 122. In this way, as a physician 24 slides the first handle 120 and the second handle 220 toward each other (as shown in FIG. 2A), the focal end effector portion 210 can extend beyond a distal end of the expandable end effector portion 110 such that the end effector 28 is configured for delivering ablative energy to, or mapping electrophysiological signals of, small areas of tissue within the heart 12. Alternatively, when the physician moves the first handle 120 and the second handle 220 away from each other (as shown in FIG. 2B), the expandable end effector portion 110 will slide beyond a distal end of the focal end effector portion 210 such that the expandable end effector portion 110 is configured to contact tissue and deliver ablative energy to, or collect electrophysiological signals from, larger areas of tissue within the heart 12. In other words, the physician 24 can slide the second handle 220 distally to move the expandable end effector portion 110 into contact with tissue to perform mapping of electrophysiological signals and the physician 24 can slide the second handle 220 proximally to expose the focal end effector portion 210 to perform an ablation procedure.

The first handle 120 can include an actuator 124 that can be configured to cause the end effector 28 to deflect or bend radially outward away from a longitudinal axis LA. The actuator 124, for example, can be attached to a pull wire that is attached to a distal end of the inner catheter shaft 222 or to the focal end effector portion 210. As the physician 24 actuates the actuator 124, the pull wire will be pulled to cause the end effector 28 to deflect outward from the longitudinal axis LA. As will be appreciated, as the inner catheter shaft 222 is deflected outwardly, the inner catheter shaft 222 will also cause the sleeve 122 (which is disposed around the inner catheter shaft 222) to deflect outwardly. In this way, both the focal end effector portion 210 and the expandable end effector portion 110 will be caused to deflect outwardly from the longitudinal axis LA when the actuator 124 is actuated.

The first handle 120 can further comprise a first irrigation coupler 126 and a first electrical coupler 128. The first irrigation coupler 126 can be configured to connect to an irrigation supply and deliver irrigation through the first handle 120 and to the expandable end effector portion 110. The expandable end effector portion 110 can include irrigation holes configured to deliver irrigation fluid to the tissue near the end effector 28. The first electrical coupler 128 can be configured to connect to a corresponding electrical coupler to connect the catheter 14 to the PIU 30 so that ablative energy supplied by the ablation energy generator 50 can be delivered to the expandable end effector portion 110 and/or so that electrical signals detected by the expandable end effector portion 110 can be analyzed and output for display.

The second handle 220 can comprise a second irrigation coupler 226 and a second electrical coupler 228. The second irrigation coupler 226 can be configured to connect to an irrigation supply and deliver irrigation fluid through the second handle 220 and to the focal end effector portion 210. The focal end effector portion 210 can include irrigation holes configured to deliver irrigation fluid to the tissue near the focal end effector portion 210. The second electrical coupler 228 can be configured to connect to a corresponding electrical coupler to connect the catheter 14 to the PIU 30 so that ablative energy supplied by the ablation energy generator 50 can be delivered to the focal end effector portion 210 and/or so that electrical signals detected by the focal end effector portion 210 can be analyzed and output for display.

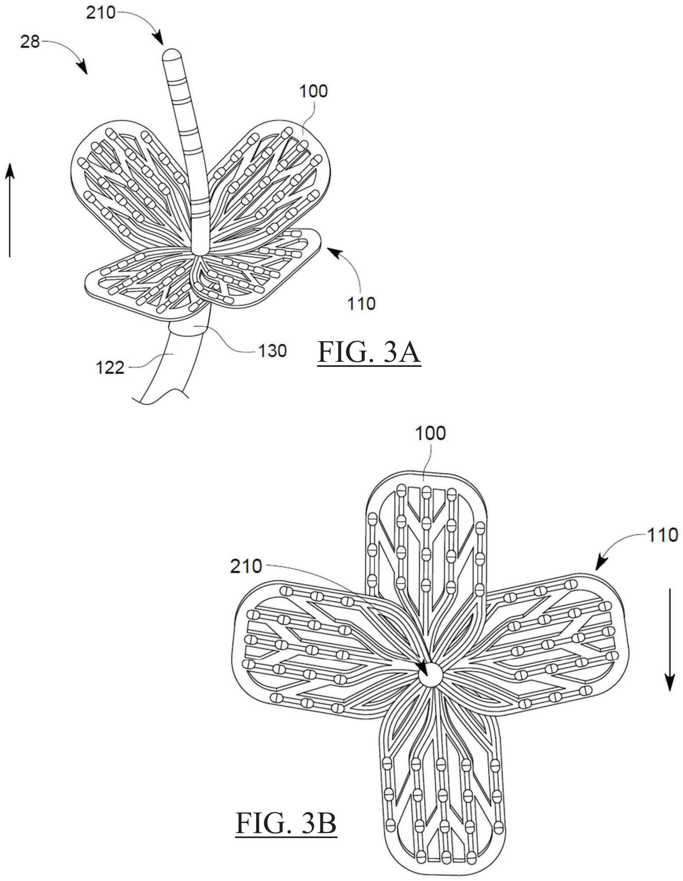

FIGs. 3A and 3B illustrate detail views of a first example of the end effector 28 with the focal end effector portion 210 being in an extended position in FIG. 3A and in a retracted position in FIG. 3B. As shown, the expandable end effector portion 110 can include a plurality of planar members 100 (in this example, four planar members 100 are shown) attached to a hub 130 that is attached to a distal end of the sleeve 122. Only a single planar member 100 is labeled in FIGs. 3A and 3B for simplicity, but it will be appreciated that the expandable end effector portion 110 can include a plurality planar members 100 extending outwardly from the hub 130. For example, as shown in FIGs. 3A and 3B, the expandable end effector portion 110 can include at least four planar members 100.

Because the planar members 100 of the expandable end effector portion 110 all extend outwardly from the hub 130, the planar members 100 can collectively be configured to contact a large area of tissue to detect electrophysiological signals and/or deliver ablative energy to tissue via electrodes. In contrast, if only a small are of tissue is intended to be ablated or mapped, the focal end effector portion 210 can pushed distally beyond the expandable end effector portion 110 and contact the tissue without the expandable end effector portion 110 contacting tissue. In this way, the end effector 28 can be used to map and ablate both small and large areas of tissue using a single device. The electrodes can be disposed on an inwardly-facing side (an inner surface member) and an outwardly-facing side (an outer surface member) of the planar member 100. The inwardly-facing side can be configured to face the focal end effector portion 210 and the outwardly-facing side can be configured to face outwardly away from the focal end effector portion 210.

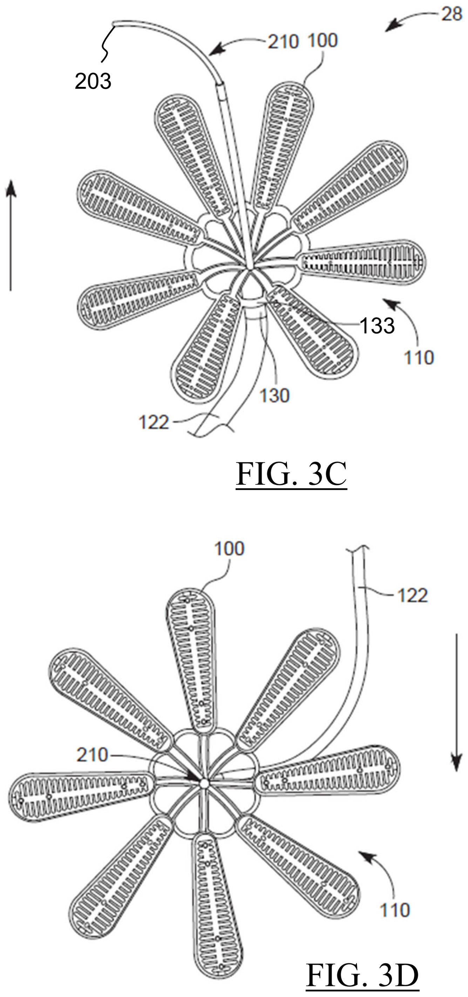

FIGs. 3C and 3D illustrate detail views of another example of the end effector 28 with the focal end effector portion 210 being in an extended position in FIG. 3C and in a retracted position in FIG. 3C. As shown in this example, the expandable end effector portion 110 can include six planar members 100 attached to a hub 130 that is attached to a distal end of the sleeve 122. Only a single planar member 100 is labeled in FIGs. 3C and 3D for simplicity. As shown, the expandable end effector portion 110 can further include support members 133 that extend between adjacent planar members 100 to provide additional support for the planar members 100 and to help keep the expandable end effector portion 110 in a predetermined shape when expanded. In the example shown in FIG. 3C, the focal end effector portion 210 can include an atraumatic tip 203 to help prevent damage to tissue.

Because the planar members 100 of the expandable end effector portion 110 all extend outwardly from the hub 130, the planar members 100 can collectively be configured to contact a large area of tissue to detect electrophysiological signals and/or deliver ablative energy to tissue. In contrast, if only a small are of tissue is intended to be ablated or mapped, the focal end effector portion 210 can pushed distally beyond the expandable end effector portion 110 and contact the tissue without the expandable end effector portion 110 contacting tissue. In this way, the end effector 28 can be used to map and ablate both small and large areas of tissue using a single device.

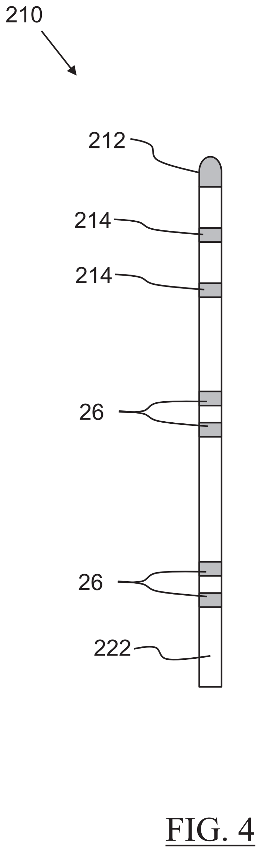

FIG. 4 illustrates a detail view of the focal end effector portion 210. As shown, the focal end effector portion 210 can include a distal electrode 212 disposed on a distal tip of the inner shaft 222, a plurality of proximal electrodes 214 disposed along the inner shaft 222 proximal of the distal electrode 212, and a plurality of impedance-based tracking electrodes 26 disposed along the inner shaft 222 proximal of the proximal electrodes 214. The distal electrode 212 and the proximal electrodes 214 can each be configured to deliver ablative energy to tissue, detect electrophysiological signals along the tissue, and/or both. In this way, the distal electrode 212 can be configured to ablate small areas of tissue and/or map electrophysiological signals along small areas of tissue.

The proximal electrodes 214 can be configured to detect electrophysiological signals along the tissue such that the inner shaft 222 can be slid along the tissue to map the electrophysiological signals. Alternatively, or in addition, the proximal electrodes 214 can be configured as a reference electrode for conducting PFA ablation with the distal electrode 212.

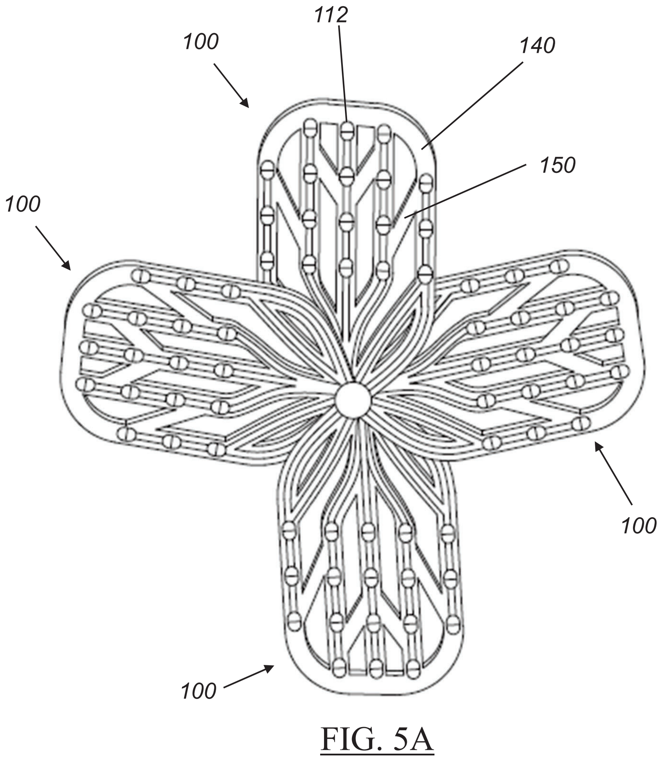

FIG. 5A is a detail view of the expandable end effector 110 showing multiple planar members 100. As shown, each planar member 100 can include a plurality of electrodes 112 disposed in spaced locations across the planar member 100 (only a single electrode 112 is labeled for the sake of simplicity). The electrodes 112 can be disposed across a planar body 140 on both a first side (as shown) of the planar body 140 and a second side of the planar body 140 opposite the first side (not shown in FIG. 5A). The electrodes 112 can be configured to deliver ablative energy to tissue. In some examples, the ablation electrodes 112 can be flush with the planar body 140 to provide a first planar surface to the planar member 100 corresponding to the first side of the planar body 140 and a second planar surface to the planar member 100 corresponding to the second side of the planar body 140.

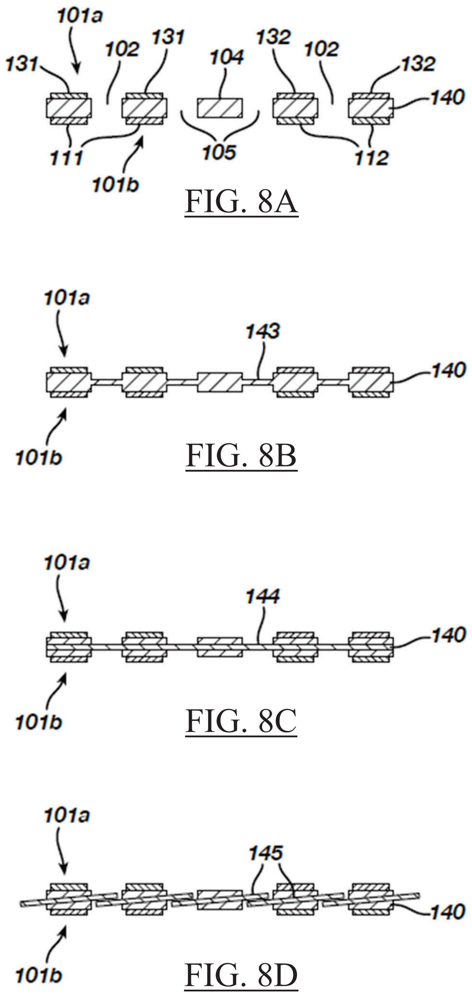

The planar body 140 includes a frame 150 surrounded by a flexible, electrically insulating encapsulation (as shown and described in greater detail in FIGs. 5B and 5C. The insulating encapsulation may be composed primarily of polymeric material (e.g., thermoplastic polyurethane) or may include more highly insulative components as described in greater detail in relation to FIGs. 8B through 8D. In this way, the electrodes on the first side can be separated by an insulative material from the electrodes disposed on the second side. The frame 150 is resilient and provides structural support for the planar member 100, allowing the expandable end effector portion 110 to be constricted to a delivery configuration for delivery through a sheath then self-expand to the unconstrained configuration as illustrated in FIGs. 1-3B, flex to allow each planar member 100 to conform to a planar or approximately planar surface such as tissue, and collapse upon retraction into a sheath. The frame 150 may be formed from a super elastic material and/or a shape-memory alloy such as nickel-titanium, also known as Nitinol, cobalt chromium, stainless steel, and/or other alloys that exhibit pseudo-elastic and/or super-elastic properties.

FIG. 5B is an illustration of a planar member 100 of the expandable end effector portion 110. The planar member 100 shown in FIG. 5B and discussed further in relation to FIGs. 5A-11 is representative of any one of the plurality of planar members 100 that can make up the expandable end effector portion 110 discussed throughout this disclosure. Thus, although a single planar member 100 or portions of a single planar member 100 are shown and described throughout FIGs. 5A-11, it will be appreciated that other planar members 100 of the expandable end effector portion 110 can include similar features.

FIG. 5B shows a first side 101a of the planar member 100. The planar member 100 has a planar body 140 with a second side 101b (FIGs. 8A-8D, 10A-10D) opposite the first side 101a. The second side 101b preferably, but not necessarily, has electrodes positioned similar to the first side 101a. When configured with similar electrode configurations on both sides 101a, 101b of the planar member 100, either side can be positioned against tissue during treatment. When one side of the planar member 100 is in contact with tissue, corresponding electrodes on the opposite side are in contact with body fluid (e.g., blood) and can act as respective reference electrodes for those electrodes in contact with tissue. Although described throughout as being a first side 101a and a second side 101b, it will be appreciated that when one of the first side 101a and the second side 101b is oriented on the expandable end effector portion 110 facing inwardly toward the inner shaft 222, it can be considered an inner surface member. Similarly, it will be appreciated that when the other of the first side 101a and the second side 101b is oriented on the expandable end effector portion 110 facing outwardly away from the inner shaft 222, it can be considered an outer surface member.

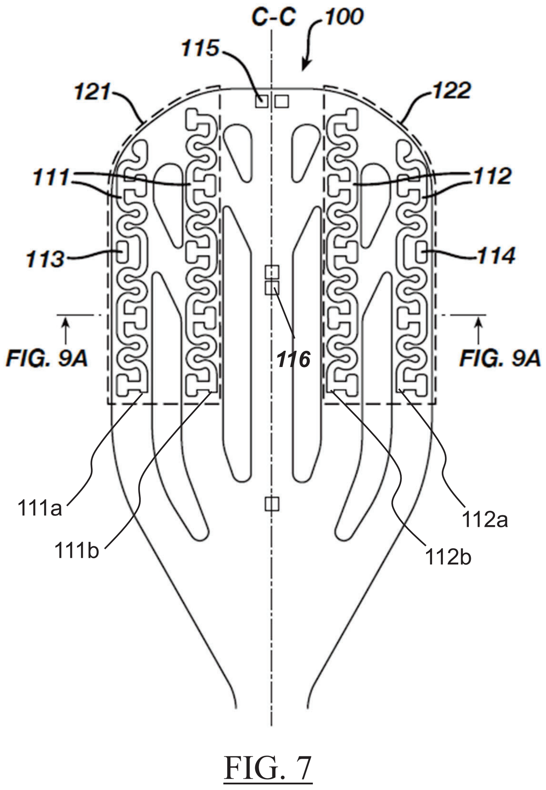

The planar member 100 includes ablation electrodes that each have longitudinally elongated segments 111a, 111b, 112a, 112b that extend along a longitudinal axis A-A defined by the catheter shaft 90. Longitudinally elongated segments 111a, 111b on a left side of the longitudinal axis A-A form a first ablation electrode 111 (FIG. 7). Longitudinally elongated segments 112a, 112b on a right side of the longitudinal axis A-A form a second ablation electrode 112 (FIG. 7). The ablation electrodes have a length L2 that is at least half of a total length L1 of the end effector 28 as measured from a distal end 106 of the end effector to a distal end of the shaft 90 of the medical probe 14. The ablation electrodes 111, 112 extend to approximately the distal end 106 of the planar member 100. The length L2 of the ablation electrodes define a distal portion of the planar member 100. The planar member 100 has a width W1 measured from leftmost and rightmost edges of the planar member 100. As illustrated, the leftmost and rightmost edges of the planar member 100 define parallel side edges of the planar member 100. The planar body 140 is preferably aligned with the longitudinal axis A-A when in an unconstrained configuration as illustrated in FIG. 5B.

The ablation electrodes 111, 112 are configured to provide ablation energy to tissue as described in greater detail elsewhere herein. The second side 101b of the planar member 100 preferably includes ablation electrodes having longitudinal elongated segments opposite the illustrated longitudinally elongated segments 111a, 111b, 112a, 112b electrodes with respect to a plane defined by the planar body 140. In some embodiments, the ablation electrodes 111, 112 are flush with the planar body 140 to provide a first planar surface to the planar member 100 corresponding to the first side 101a of the planar body 140 and a second planar surface to the planar member 100 corresponding to the second side 101b of the planar body 140.

In some embodiments, the ablation electrodes on the second side 101b can function as respective reference electrodes for the respective opposite ablation electrode on the first side 101a when the first side 101a is in contact with tissue, and vice versa. Additionally, or alternatively, the ablation electrodes on opposite sides 101a, 101b can be paired to provide bipolar PFA electrical signals between the paired ablation electrodes. Additionally, or alternatively, ablation electrodes on the same side can be paired to provide bipolar PFA electrical signals between the paired ablation electrodes. The bipolar PFA electrical signals may be monophasic or biphasic.

The illustrated planar member 100 includes optional diagnostic electrodes 113, 114 on the first side 101a that are electrically isolated from the ablation electrode segments 111a, 111b, 112a, 112b. The diagnostic electrodes 113, 114 are configured to receive electrical signals from tissue to map cardiac tissue and detect arrhythmia. As illustrated, the diagnostic electrodes 113, 114 are arranged as a pair with a first diagnostic electrode 113 on the left side of the longitudinal axis A-A and a second diagnostic electrode 114 on a right side of the longitudinal axis A-A. The diagnostic electrodes 113, 114 are disposed diametrically along an axis D-A that is orthogonal to the longitudinal axis A-A with respect to each other. The second side 101b of the planar member 100 preferably includes a second pair of diagnostic electrodes opposite the first pair of diagnostic electrodes with respect to a plane defined by the planar body 140.

The illustrated planar member 100 includes optional tissue contact quality electrodes 115, 116 positioned in closely spaced pairs such that impedance measured across the respective tissue contact quality electrode pair indicates that electrodes of that pair are both in contact with tissue. As illustrated, the first side 101a includes a distal pair of tissue contact quality electrodes 115 disposed approximate the distal end 106 of the planar member 100 and a central pair of tissue contact quality electrodes 116 positioned approximately in the middle of the length L2 of the distal portion of the planar member 100. The pairs of tissue contact quality electrodes 115, 116 are spaced apart along the longitudinal axis A-A. The planar body 140 of the end effector includes a center portion 104 extending along the longitudinal axis A-A and centrally along the total length L1 that lacks any ablation electrode. As illustrated, the tissue contact quality electrodes 115, 116 are positioned in this central portion 104. The second side 101b of the planar body 140 of the planar member 100 preferably includes corresponding tissue contact quality electrode pairs opposite the pairs of tissue contact quality electrodes 115, 116 on the first side 101a with respect to a plane defined by the planar body 140.

The illustrated planar member 100 includes an optional reference electrode 117 disposed on the first side 101a of the planar body 140. Additionally, or alternatively, the planar member 100 may include a reference electrode similarly disposed on the second side 101b of the planar body 140. The reference electrode(s) 117 may be used for ECG gathering in either, unipolar, bipolar (split or close pair) or may be a reference. The number can be as few as 4 per side but as many as needed (and can fit with trace limitations). The signals from the reference electrode(s) 117 may also be used for contact information to determine what portion of the paddle has contact or proximity to the tissue.

The planar member 100 includes a proximal portion defined by a proximal length L3 which lacks any ablation electrode. The reference electrode 117 may be disposed in this proximal portion. As discussed herein, a ablation electrode, diagnostic electrode, or tissue contact electrode may be used as a reference electrode for a corresponding electrode on the opposite side in contact with tissue. Each of these electrodes are positioned in the distal portion of the planar member 100 defined by the ablation electrode length L2 so that they may be positioned in contact with tissue if so desired by the physician 24 (FIG. 1). Preferably, the reference electrode 117 is positioned such that the physician 24 is unable, or at least very unlikely, to position the reference electrode 117 in contact with tissue but nevertheless in relatively close proximity to electrodes which are configured to contact tissue. For instance, the planar member 100 may be configured to flex such that while the distal portion of the first side 101a is in contact with tissue, a majority of the proximal portion is not in contact with tissue. By being positioned in the proximal portion, the reference electrode 117 is therefore unable or unlikely to contact tissue during a procedure. Said another way, the end effector is configured to flex such that a majority of the first side 101a of the distal portion is configured to conform to a planar surface while the reference electrode is separated from the planar surface. In such embodiments, the illustrated reference electrode 117 is a dedicated reference electrode rather than a multi-purpose electrode.

In some embodiments, at least the ablation electrodes, diagnostic electrodes, and tissue contact electrodes are flush with the planar member 100 to provide a first planar surface to the planar member 100 corresponding to the first side 101a of the planar body 140 and a second planar surface to the planar member 100 corresponding to the second side 101b of the planar body. The ablation electrodes 111, 112 and any combination of other electrodes 113, 114, 115, 116, 117 may include an exposed conductive layer in a flexible printed circuit board, may include silver epoxy, and/or conductive ink.

The illustrated planar body 140 includes openings 102, 105 between the first side 101a and the second side 101b. The openings 102, 105 can be sized, shaped, or otherwise configured to allow the end effector to flex through a sheath catheter. The openings 102, 105 can be sized, shaped, or otherwise configured to collapse or expand when the end effector is pressed against a non-planar tissue surface to provide conformal contact to the non-planar tissue surface. Applicants recognize that consistent contact of ablation electrodes to the non-planar tissue may produce improved lesions compared to less conformal electrode contact. In some embodiments the distal end may not be fully closed to allow for more flexibility and larger area. In some embodiments EM loops extend through paddle spines between the openings. An outer opening 102 is disposed between elongated ablation electrode segments 111a, 111b, 112a, 112b of respective ablation electrodes 111, 112. An inner opening 105 is disposed between inner elongated ablation electrode segments 111b, 112b and the central portion 104.

FIG. 5C is an illustration of a magnified view of a portion of the example planar member 100 as indicated in FIG. 5B. The planar body 140 includes a frame 150 surrounded by a flexible, electrically insulating encapsulation 142. The insulating encapsulation may be composed primarily of polymeric material (e.g., thermoplastic polyurethane) or may include more highly insulative components as described in greater detail in relation to FIGs. 8B through 8D. In this way, the electrodes on the first side 101a can be separated by an insulative material from the electrodes disposed on the second side 101b. The frame 150 is resilient and provides structural support for the planar member 100, allowing the expandable end effector portion 110 to be constricted to a delivery configuration for delivery through a sheath then self-expand to the unconstrained configuration as illustrated in FIGs. 1-3B, flex to allow each planar member 100 to conform to a planar or approximately planar surface such as tissue, and collapse upon retraction into a sheath. The frame 150 may be formed from a super elastic material and/or a shape-memory alloy such as nickel-titanium, also known as Nitinol, cobalt chromium, stainless steel, and/or other alloys that exhibit pseudo-elastic and/or super-elastic properties.

The illustrated portion of the planar body 140 is divided into three segments: an outer segment including an outer elongated ablation electrode segment 111a, an inner segment including an inner elongated ablation electrode segment 111b, and the central portion 104. The planar body 140 includes a second outer segment and a second inner segment on the right side of the planar body shown in FIG. 5B but not FIG. 5C so that the planar body 140 has a total of five segments. The outer segment has a width W4; the inner segment has a width W3; and the central portion 104 has a width W2. In the illustrated example, the body segment widths W2, W3, W4 are approximately equal to each other. Each elongated ablation electrode segment 111a, 111b has a width W5 that is more than half, approximately 80% of the width of the outer body segment width W4 and the inner body segment width W3. The ablation electrode segment width W5 is approximately 10% of the total width W1 of the planar member 100.

The elongated ablation electrode segments 111a, 111b are positioned left or right of each other. Each of the elongated ablation electrode segments 111a, 111b extends distally to approximately a distal edge of the planar body 140. Because the distal edge is curved, the inner ablation electrode segment 111b defines the length L2 of the distal portion, while the length L5 of the outer ablation electrode segment 111a is shorter than the length L2 of the inner ablation electrode segment 111b.

The illustrated diagnostic electrode 113 is positioned on the outer segment of the planar body 140 and entirely within the width W5 and length L5 of the outer longitudinally extending ablation electrode segment 111a. The diagnostic electrode 113 has a width W7 that is less than half, preferably approximately one third, the width W5 of the outer longitudinally extending ablation electrode segment 111a. The diagnostic electrode 113 has a length L4 less than half, less than or about one fifth, or less than or about one tenth, the length L5 of the outer longitudinally extending ablation electrode segment 111a.

Each elongated ablation electrode segment 111a, 111b has a serpentine shape. The serpentine shape of the outer longitudinally extending ablation electrode segment 111a bends around three of the four sides of the diagnostic electrode 113. As illustrated, the serpentine shape has a path width W6 that is approximately equal to the width W7 of the diagnostic electrode W7 and approximately one third of the width W5 of the outer longitudinally extending ablation electrode segment 111a.

The planar member 100 further includes electrical traces 118, 119 having an insulated, serpentine-shaped electrical conductor portion. The illustrated electrical traces 118, 119 extend longitudinally, forming a serpentine path within the central portion 104. The illustrated electrical traces 118, 119 make electrical connection to the central pair of tissue contact quality electrodes 116 and the distal pair of tissue contact quality electrodes 115.

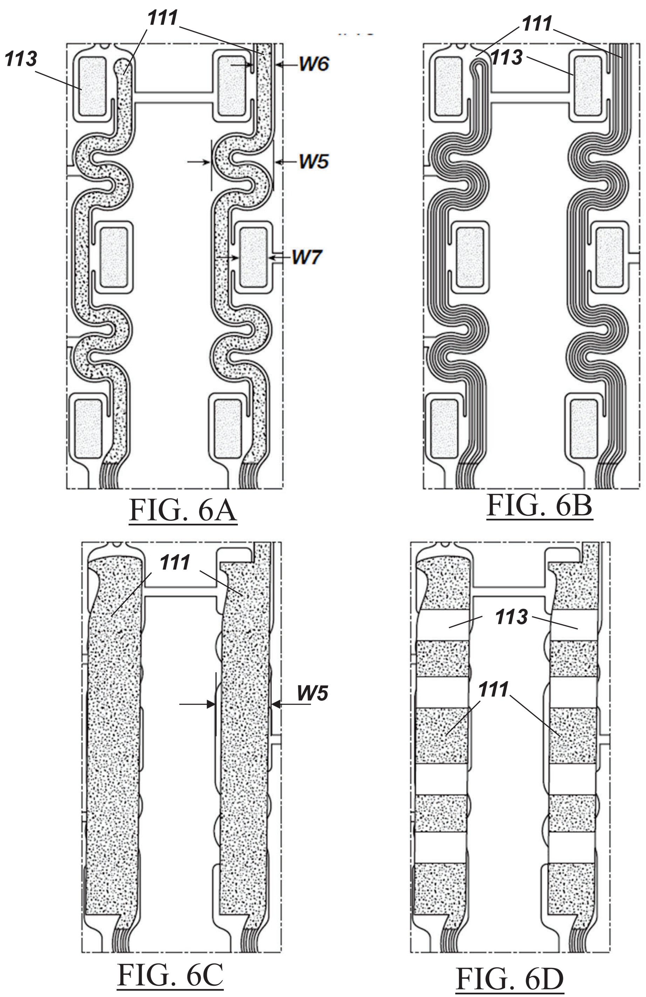

FIG. 6A is an illustration of a first alternative electrode configuration for the example planar member 100 including one example configuration of a ablation electrode 111 and diagnostic electrodes 113. Similar to FIGs. 5B and 5C, the ablation electrode 111 includes serpentine longitudinally extending segments with a total width W5 and a path width W6. While the configuration in FIGs. 5B and 5C includes only a single diagnostic electrode 113 per ablation electrode 111, the example in FIG. 6A includes six diagnostic electrodes 113 per ablation electrode 111. The planar member 100 may include one, two, three, four, five, or six diagnostic electrodes 113 per ablation electrode 111. Each diagnostic electrode is similarly sized with a width W7 that is approximately one and a half times the path width W6 of the ablation electrode 111 and approximately one half the total width W5 of an elongated segment of the ablation electrode 111. The diagnostic electrodes 113 are surrounded on two or three sides by a respective elongated segment of the ablation electrode 111.

FIG. 6B is an illustration of a second alternative electrode configuration for the example planar member 100 including ablation electrodes 111 having elongated segments which are respectively segmented into small parallel stripes and diagnostic electrodes 113 positioned alongside the elongated ablation electrode segments. Each of the elongated segments has multiple electrically conductive stripes running parallel to each other to form an overall shape of a respective elongated segment that is similar to as shown in FIG. 6A. Other ablation electrode shapes illustrated herein, and alternatives thereto as understood by a person skilled in the pertinent art may also be divided into multiple electrically conductive stripes running parallel to each other similar to as shown in FIG. 6B. This configuration increases the total edge length of the ablation electrode to provide a different electric field profile to tissue during PFA than a similarly shaped ablation electrode that is solid (e.g., as shown in FIG. 6A).

FIG. 6C is an illustration of a third example electrode configuration for the example end effector including rectangular elongated segments of the ablation electrode 111 without diagnostic electrodes 113. The entire width W5 of the electrode segment is utilized for the ablation electrode 111 to maximize surface area of the ablation electrode 111. Diagnostic electrodes may be disposed elsewhere on the planar body, for instance in the central portion 104.

FIG. 6D is an illustration of a fourth example electrode configuration for the example end effector including rectangular elongated segments of the ablation electrode 111 interrupted by diagnostic electrodes 113. Compared to the continuous rectangular electrode segments shown in FIG. 6C, the segmented elongated ablation electrode segments can have greater flexibility for the planar body 140 to flex away from the longitudinal axis A-A at the expense of total ablation electrode area.

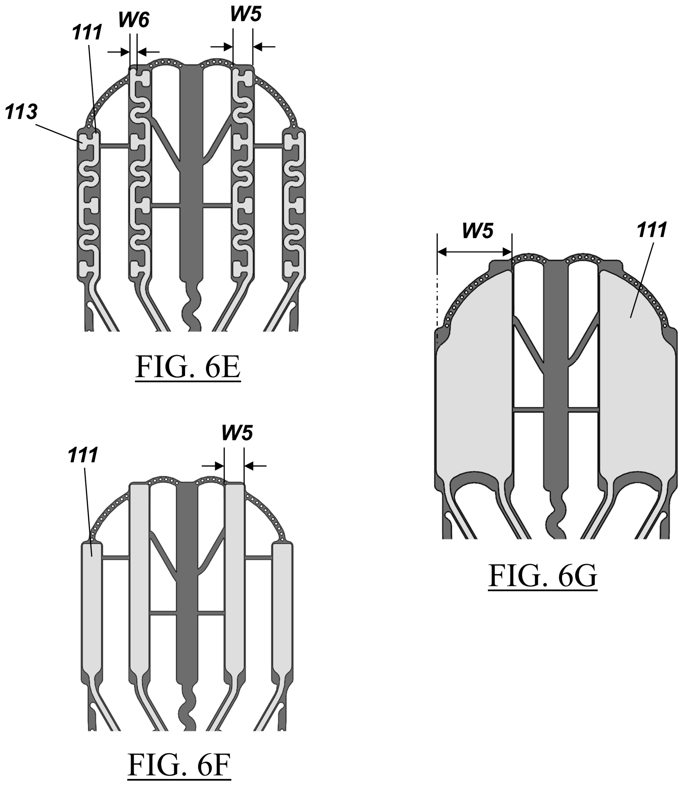

FIG. 6E is an illustration of an example electrode configuration for the example planar member 100 including elongated serpentine ablation electrodes 111. The elongated segments are serpentine similar to as illustrated in FIG. 6A. In one embodiment, the trace width W6 is approximately 0.28 mm, the electrode segment width W5 is approximately 1 mm, the ablation electrode surface area is approximately 10.1 square mm, the perimeter is approximately 67.7 mm, and the perimeter to area ratio is approximately 7:1.

FIG. 6F is an illustration of an example electrode configuration for the example end effector including rectangular elongated ablation electrodes 111. The elongated segments are solid similar to as illustrated in Figure 6C, meaning the trace width is equal to the total width. In one embodiment, the electrode segment width W5 is approximately 1 mm, the ablation electrode surface area is approximately 16.8 square mm, the perimeter is approximately 37.4 mm, and the perimeter to area ratio is approximately 2:1.

FIG. 6G is an illustration of an example electrode configuration for the example end effector including large area ablation electrodes 111. In one embodiment, the electrode segment width W5 is approximately 3.5 mm, the ablation electrode surface area is approximately 28.8 square mm, the perimeter is approximately 24.8 mm, and the perimeter to area ratio is approximately 1:1

FIG. 7 is an illustration of an example electrode configuration on one side the example planar member 100 including elongated ablation electrodes 111, 112 in two ablation electrode regions 121, 122, diagnostic electrodes 113, 114 positioned in the two ablation electrode regions 121, 122, tissue contact electrodes 115, 116 positioned between the two ablation electrode regions 121, 122, and a reference electrode 117 positioned in a proximal region of the illustrated side of the planar member 100. In some embodiments, the total area of each ablation electrode 111, 112 within a respective electrode region 121, 122 is approximately 7.5 mm². In FIG. 7, it can be seen that there are two individual ablation electrodes 111a, 111b on the left side and two individual ablation electrodes 112a, 112b on the right side. Left side electrodes 111a and 111b can be connected to the pulse field generator to deliver a biphasic pulse field between the electrodes 111a and 111b. Right side electrodes 112a and 112b can be connected to the pulse field generator to deliver a biphasic pulse field between the electrodes 112a and 112b. Alternatively, each of the left electrodes 111a and 111b can be connected to the generator with the each of the right electrodes 112a and 112b to deliver a bipolar pulse between the left side electrode and the right side electrode. For example, a bipolar electrode pair can be formed by one or more left electrode 111a and 111b with one or more right electrodes 112a and 112b.

A centerline C-C is drawn in FIG. 7 which indicates a line of symmetry for an electrode configuration and/or planar member 100. As illustrated, the electrode configuration is symmetric about a centerline C-C. Preferably, at least the ablation electrodes 111, 112 are symmetric to each other with respect to the centerline C-C. The planar member 100 may also be a symmetric shape about the centerline C-C as illustrated. In an alternative embodiment, the planar member 100 may not be symmetric about the centerline C-C.

The illustrated side of the planar member 100 has a left half which is left of the centerline C-C and a right half which is right of the centerline C-C. The first ablation electrode 111 defines the first electrode region 121, which is entirely in the left half of the planar member 100. The second ablation electrode 112 defines the second electrode region 122, which is entirely in the right half of the planar member 100. The tissue contact electrodes 115, 116 are arranged in pairs that each are disposed across the centerline C-C on the illustrated side of the planar member 100. A distal pair of tissue contact electrodes 115 are approximate the distal end 106 of the planar member 100. A central pair of tissue contact electrodes 116 are in a proximal direction in relation to the distal pair 115 and across the centerline C-C. The reference electrode 117 is disposed on the illustrated side of the planar member 100 and across the centerline C-C.

FIGs. 8A through 8D are illustrations of alternative configurations of insulating material in the planar body 140 cross-section. In each figure, a second side 101b of the planar body 140 is indicated opposite the first side 101a. Ablation electrodes 131, 132 are illustrated on the second side 101b opposite the ablation electrodes 111, 112 on the first side. The opposite ablation electrodes 131, 132 overlap the ablation electrodes 111, 112 on the first side 101a, preferably overlapping a majority of the ablation electrodes 111, 112 on the first side 101a, and may be symmetric to the ablation electrodes 111, 112 on the first side 101a with respect to a plane defined by the planar member 100. The cross-section of the planar member 100 is simplified to omit the frame 150, electrical traces, and other such features for the sake of illustration. Further, the ablation electrodes 111, 112, 131, 132 are labeled only in FIG. 8A for simplicity, but it will be appreciated that the ablation electrodes 111, 112, 131, 132 are similarly positioned in FIGs. 8B-8D.

The ablation electrodes 111, 112, 131, 132 are illustrated projecting from the planar body 140, but may alternatively be sunken into the planar body 140 to provide a smooth planar surface on each respective side 101a, 101b. The ablation electrodes 111, 112, 131, 132 can have approximately equal surface area with respect to each other. Each segment 111a, 111b, 112a, 112b, 131a, 131b, 132a, 132b of the ablation electrodes are shown as contiguous across their width. Alternatively, some or all of the segments can include parallel stripes similar to as illustrated in FIG. 6B, resulting in a segmented cross-sectional profile.

The various configurations shown in FIGs. 8B through 8D provide differing levels of electrical insulation between electrodes on opposite sides of the planar body 140. The electrical insulation may be tailored to direct electric field lines, and thereby electroporation of cells within target tissue, between bipolar pairs of ablation electrodes on opposite sides of the planar body 140 during PFA application.

FIG. 8A is configured with openings in the end effector as indicated in FIG. 5B. FIG. 8B includes a polymeric body region 143 forming an insulative material extending a width of planar body 140. Essentially, some or all of the openings 102, 105 (and other non-labeled openings) of the example planar member 100 illustrated in FIGs. 5B and 5C can include a respective thinned polymer fill-in region which lacks a framework or any electrical circuitry. Configured as such, the planar body 140 may provide additional electrical insulation between electrodes on opposite sides of the planar body 140 (compared to openings 102, 105) while maintaining sufficient flexibility for manipulation to position against tissue and transfer through a sheath.

FIG. 8C includes a planar high dielectric layer 144 disposed between each side and extending a width of the end effector. The dielectric layer 144 is overlapping and parallel to each of the ablation electrodes 111, 112, 113, 114. The planar high dielectric layer 144 can include a ceramic doped polymer to provide additional electrical insulation between electrodes on opposite sides of the planar body compared to polymer alone while still providing sufficient flexibility. As illustrated, the planar high dielectric layer 144 extends across a majority of an entire width W1 of the planar body 140. The planar body 140 includes longitudinally elongated regions (e.g. corresponding to openings 102, 105 in FIGs. 5B and 5C) including the planar high dielectric layer 140, lacking a framework, and lacking electrical circuitry.

FIG. 8D includes high dielectric tiles 145 between each side 101a, 101b and configured to overlap to collapse the planar member 100 into a delivery sheath. The high dielectric tiles 145 can include ceramic plates that extend longitudinally through each segment of the body and overlap between segments such that the overlap corresponds to openings 102, 105 illustrated in FIGs. 5B and 5C. The high dielectric tiles or longitudinally extending ceramic plates may be angled with respect to a plane defined by the planar body 140 such that the tiles/plates 145 are configured to overlap, longitudinal side on longitudinal side upon retraction of the end effector into a sheath.

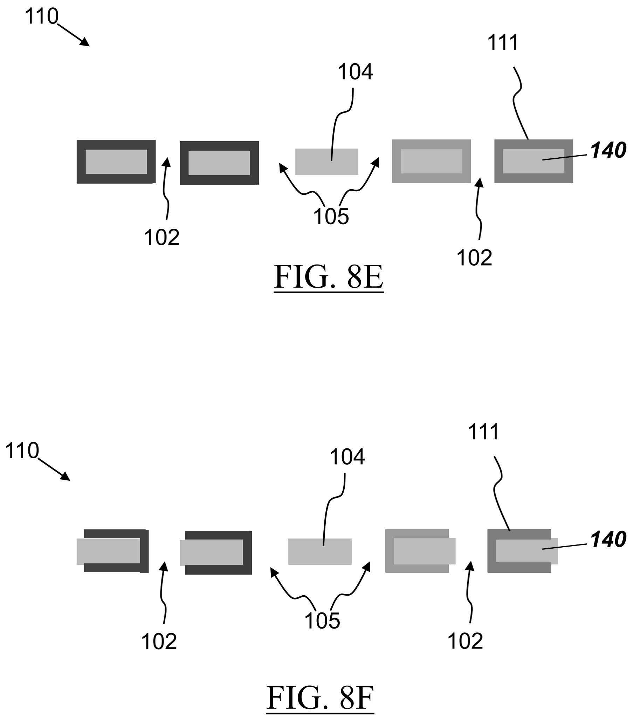

FIG. 8E is an illustration of an example electrode 111 configuration for the planar member 100 in which ablation electrodes 111 wrap around a spine or insulative material of the planar body 140. FIG. 8F is an illustration of an example electrode 111 configuration for the planar member 100 in which ablation electrodes 111 partially wrap around a spine or insulative material of the planar body 140. Each electrode in FIGs. 8E and 8F can be activated separately to form bipolar pairs symmetric or asymmetric with respect to the centerline C-C similar to the examples further discussed herein.

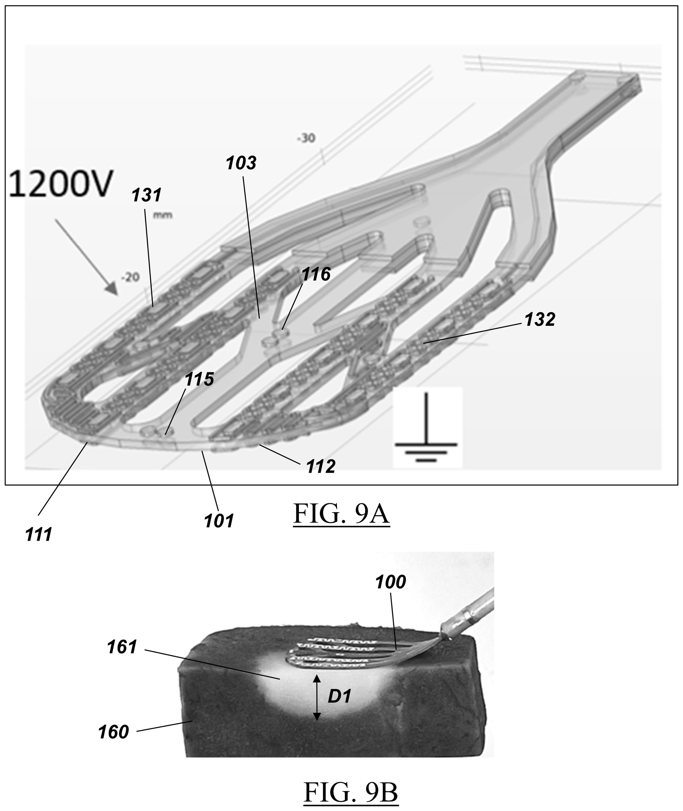

FIG. 9A is an illustration of the end planar member 100 in which the ablation electrodes are configured to provide 1,200 V pulses between ablation electrode regions. In some embodiments, the planar member 100 is configured to provide PFA electrical pulses having a voltage of about 600 V and about 2,600 V between pairs of ablation electrodes 111, 112, 131, 132. As illustrated in FIG. 9A, the planar member 100 is configured to provide electrical pulses between about 600 V and about 2,600 V in amplitude between bipolar pairs which include a ablation electrode on the left side of the planar member 100 in contact with tissue, i.e., electrode 111 and an electrode on the right side of the planar member 100 and not in contact with tissue, i.e., electrode 132. Similarly, electrodes 112 and 131 are paired. The paired electrodes are on opposite sides of the planar body 140 and non-overlapping.

In some embodiments, cardiac electrical signals may be measured from one or more diagnostic electrode(s) disposed on the side of the planar member 100 in contact with tissue. In some embodiments, tissue contact may be measured from a pair of tissue contact electrodes disposed on the side of the planar member 100 in contact with tissue. In some embodiments, a reference electrical signal may be measured from a reference electrode disposed on the side of the planar member 100 in contact with tissue, wherein the reference electrode itself is not in contact with tissue.

FIG. 9B is an illustration of the planar member 100 applying the treatment illustrated in FIG. 9A to a potato model 160. The PFA electrical signals applied to the ablation electrodes results in a lesion 161 having a depth D1 of approximately 7.5 millimeters (mm). To create the lesion, a first bipolar PFA electrical signal is applied a first ablation electrode and a second ablation electrode, the first ablation electrode being disposed on a first side of a planar body of the planar member 100, and the second ablation electrode being disposed on a second side of the planar member 100 and non-overlapping with the first ablation electrode (i.e. ablation electrode pair 111, 132). The first bipolar PFA electrical signal includes 60 pulses with a magnitude of approximately between about 600 V and about 2,600 V and a total duration of approximately 4 seconds. A second bipolar PFA electrical signal is applied between a third ablation electrode and a fourth ablation electrode, the third ablation electrode being disposed on a first side of a planar member 100 and overlapping with the second ablation electrode, and the fourth ablation electrode being disposed on a second side of the planar member 100 and overlapping with the second ablation electrode, i.e., ablation electrode pair 112, 131. The second bipolar PFA electrical signal includes 60 pulses with a magnitude of approximately between 600 V and about 2,600 V and a total duration of approximately 4 seconds.

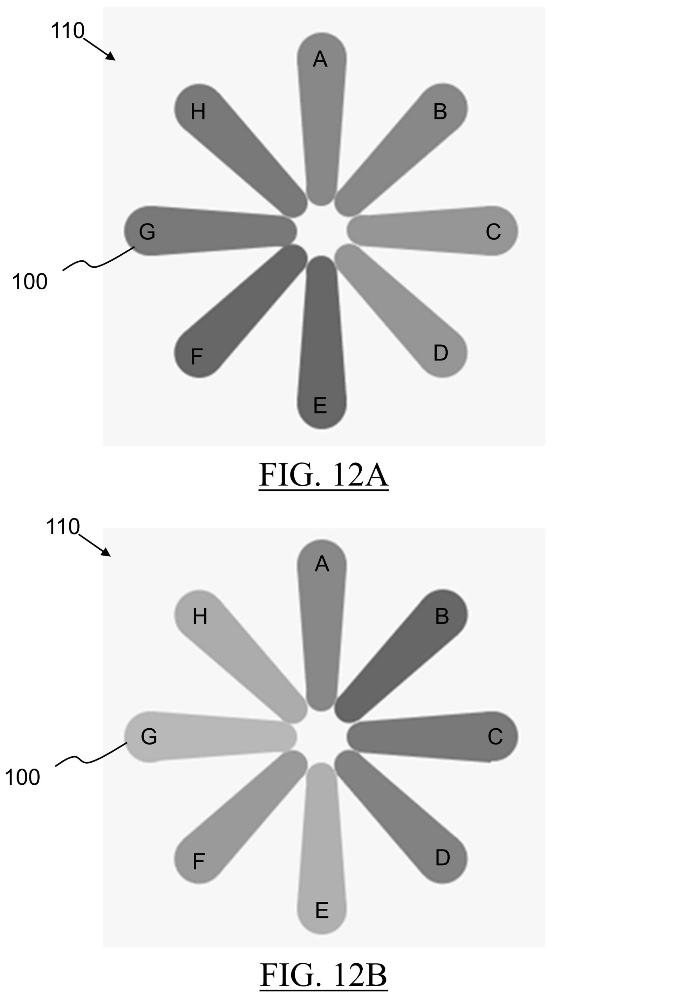

FIGs. 12A, 12B, 13, and 14 illustrate ablation electrode pairing configurations for application of bipolar PFA electrical pulses having a voltage of about 600 V and about 2,600 V in embodiments in which the planar member 100 includes a total four ablation electrodes 111, 112, 131, 132. As described in greater detail in relation to FIG. 9A, the planar member 100 preferably includes a total of two to eight ablation electrodes. The pattern of ablation electrode pairings can be adapted based on the total number of ablation electrodes in the planar member 100 as understood by a person skilled in the pertinent art informed by the disclosure herein.

FIGs. 10A and 10B illustrate a cross-body pairing to ablation electrodes as described in relation to FIGs. 9A and 9B. Ablation electrodes in a pair are symmetric with respect to the centerline C-C (illustrated in FIG. 7) and on opposite sides of the planar body 140. A voltage is applied across electrodes in pairs of electrode regions in which, for each pair, ablation electrodes in a first electrode region is in contact with tissue and the other electrode region is on an opposite side of the planar member 100 and across the center line C-C from the first electrode region.

FIGs. 10A and 10B show two different pairs of electrode regions. FIG. 10A illustrates the elongated segments 111a, 111b of ablation electrode 111 having a positive charge while the elongated segments 132a, 132b of ablation electrode 132 have a negative charge. This illustrates a positive voltage pulse configured to induce electroporation in tissue in contact with either side 101a, 101b of the planar body 140. A biphasic pulse may be applied in which the polarity switches or alternates between as shown in FIG. 10A to one in which positive charge is on the ablation electrode 132 not in contact with tissue while negative charge is on the ablation electrode 111 in contact with tissue. A train of bipolar pulses (which may include biphasic and/or monophasic pulses) can be applied between the bipolar ablation electrode pair 111, 132 as shown in FIG. 10A, then subsequently, a train of bipolar pulses can be applied between the bipolar ablation electrode pair 112, 131 shown in FIG. 10B.

FIG. 10C illustrates ablation electrode pairing configuration in which ablation electrodes in the pair are symmetric with respect to the center line C-C (illustrated in FIG. 7) and on the same side of the planar body 140. As illustrated, a voltage is applied across electrodes 111, 112 on the side 101a of the planar body 140 in contact with tissue. The electrodes 131, 132 not in contact with tissue may function as reference electrodes. A biphasic pulse may be applied in which the polarity switches or alternates between as shown in FIG. 10C to one in which positive charge is on the right ablation electrode 112 while negative charge is on the left ablation electrode 111. A train of bipolar pulses can be applied between the bipolar ablation electrode pair 111, 112.

FIG. 10D illustrates a ablation electrode pairing configuration in which ablation electrodes in the pair are overlapping and on opposite sides of the planar body. A voltage is applied across ablation electrodes 111, 112 in contact with tissue to ablation electrodes 131, 132 on an opposite side of the planar body 140 and not in contact with tissue. A biphasic pulse may be applied in which the polarity switches or alternates between as shown in FIG. 10D to one in which positive charge is on ablation electrodes 131, 132 not in contact with tissue while negative charge is on ablation electrodes 111, 112 in contact with tissue. A train of bipolar pulses can be applied between the bipolar ablation electrode pairs. As illustrated, both electrodes 111, 112 in contact with tissue are activated simultaneously. Alternatively, a train of bipolar pulses may be applied to the first pair of ablation electrodes while the second pair is deenergized, and subsequently a train of bipolar pulses may be applied to the second pair of ablation electrodes while the first pair is deenergized.

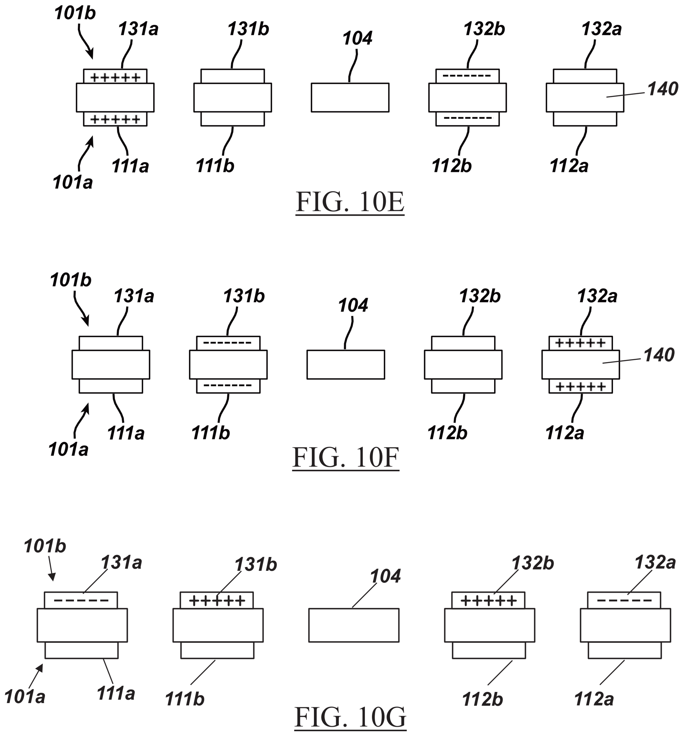

FIGs. 10E and 10F illustrate a voltage applied across electrode pairs in which electrodes of the pair are positioned across the central axis from each other. The pairs are asymmetric with respect to the centerline such that one electrode (or set of electrodes) in the pair is closer to the centerline than the other. In this configuration, the electrode segments on opposite sides of the end effector body are activated as a unit and may be electrically coupled within the planar member 100. For example, electrodes 111a and 131a can form a bipolar pair with electrodes 112b and 132b as shown in FIG. 10E. Similarly, as shown in FIG. 10F, electrodes 111b and 131b can form a bipolar pair with electrodes 112a and 132a.

Alternatively, although not shown, only electrodes on a side of the planar member 100 in contact with tissue may be activated. For instance, ablation electrodes 111a and 112b may form a bipolar pair in FIG. 10E while opposite ablation electrodes 131a and 132b are off. Likewise, in FIG. 10F, ablation electrodes 111b and 112a may form a bipolar pair in FIG. 10E while opposite ablation electrodes 131b and 132a are off. A train of bipolar pulses (which may include biphasic and/or monophasic pulses) can be applied between bipolar ablation electrode pairs as shown in FIG. 10E and subsequently in FIG. 10F, switching back and forth between the bipolar configurations in FIGs. 10E and 10F.

FIG. 10G illustrates another example wherein the ablation electrodes 131a and 131b considered to be in contact with tissue. In this example, ablation electrodes 131a and 131b are no longer connected to each other but are separately connected to the generator so that a biphasic pulse field can be provided between these two tissue contacting ablation electrodes to allow the flow of electrons between 131a and 131b. Similarly, ablation electrodes 131c and 131d (in tissue contact) are no longer connected to each other but are separately connected to the generator so that a biphasic pulse field can be provided between these two tissue contacting ablation electrodes to allow the flow of electrons between them.

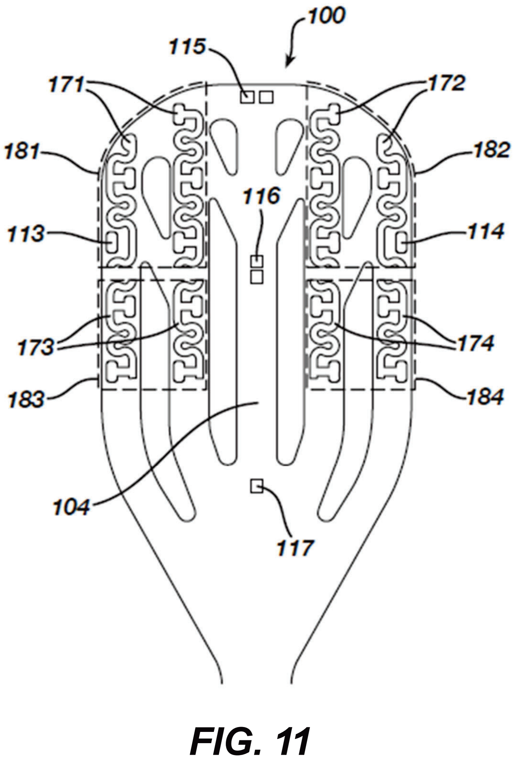

FIG. 11 is an illustration of an example electrode configuration on one side the example planar member 100 including elongated ablation electrodes 171, 172, 173, 174 in four ablation electrode regions 181, 182, 183, 184, diagnostic electrodes 113, 114 positioned in at least two of the ablation electrode regions 181, 182, tissue contact electrodes 115, 116 positioned along the centerline C-C between ablation electrode regions, and a reference electrode 117 positioned in a proximal region of the illustrated side of the planar member 100. While embodiments of the planar member 100 described herein above include exactly four ablation electrodes 111, 112, 131, 132; the embodiment illustrated in FIG. 11 includes up to eight ablation electrodes including those illustrated 171, 172, 173, 174 and corresponding overlapping ablation electrodes on the opposite side of the planar body not illustrated. It will be appreciated, however, that the planar member 100 can include just one ablation electrode or it can include more than 8 ablation electrodes depending on the particular configuration.

The planar member 100 preferably includes a total of two to eight ablation electrodes. The planar member 100 preferably includes exactly two, three, or four ablation electrodes per side of the planar body 140. Each ablation electrode preferably overlaps a corresponding ablation electrode on the opposite side of the planar body so that the ablation electrodes are symmetric with respect to a plane defined by the planar body 140. Fewer ablation electrodes can be accomplished by electrically connecting combinations of ablation electrodes within the planar member 100 or elsewhere within the catheter 14. Increasing the number of ablation electrodes can be accomplished by splitting apart a ablation electrode into two portions that are electrically insulated from each other in the catheter 14 and configured to be independently activated by the generator 50. FIG. 11 shows one example in which the ablation electrode 111 on the left of the centerline C-C as shown in FIG. 7 is segmented into two electrodes: an upper left ablation electrode 171 and a lower left ablation electrodes 173. Likewise, the ablation electrode 112 on the right of the centerline C-C as shown in FIG. 7 is segmented into two electrodes: an upper right ablation electrode 172 and a lower right ablation electrode 174. Preferably, all ablation electrodes 171, 172, 173, 174 have approximately the same surface area as each other.

Ablation electrodes can be paired in various pairing combinations to provide bipolar PFA electrical signals between ablation electrodes in a pair. Table 1 includes a summary of selected example ablation electrode pairings based on the illustrated examples herein. Pairs can be activated simultaneously and/or sequentially in various combinations to achieve PFA of target tissue as understood by a person skilled in the pertinent art informed by the disclosure herein.

**Table 1 Summary of Selected Example Ablation electrode Pairings**

| **Ablation electrode pairing** | **Examples (reference numbers of ablation electrodes in a bipolar pair with "opposite" indicating electrode on the opposite side of planar body than what is illustrated in** **FIG. 11****)** |
|---|---|
| Symmetrically across the center line C-C and on opposite sides of the planar body | (111, 132) in FIG. 10A; (112, 131) in FIG. 10B; (171, opposite 172) in FIG. 11; (173, opposite 174) in FIG. 11; (172, opposite 171) in FIG. 11; and (174, opposite 173) in FIG. 11 |
| Symmetrically across the center line C-C and on same side of the planar body | (111, 112) in FIG. 10C; (131, 132) opposite the planar body as shown in FIG. 10C (when in contact with tissue); (171, 172) in FIG. 11; and (173, 174) in FIG. 11 |
| Overlapping and on opposite sides of the planar body | (111, 131) in FIG. 10D; (112, 132) in FIG. 10D; (171, opposite 171) in FIG. 11; (172, opposite 172) in FIG. 11; (173, opposite 173) in FIG. 11; (174, opposite 174) FIG. 11 |
| On the same side of the center line C-C and on the same side of the planar body | (171, 173); and (172, 174) in FIG. 11 |
| On the same side of the center line C-C, non-overlapping, and on opposite sides of the planar body | (171, opposite 173); (173, opposite 171); (172, opposite 174); and (174, opposite 172) in FIG. 11 |
| Asymmetrically across the center line C-C and on opposite sides of the planar body | (171, opposite 174); (173, opposite 172); (172, opposite 173); and (174, opposite 171) in FIG. 11 |
| Asymmetrically across the center line C-C and on the same side of the planar body | (171, 174); and (172, 173) in FIG. 11 |

In another alternative embodiment (not illustrated), the segments 111a, 111b of the left ablation electrode 111 illustrated in FIG. 5B may be electrically insulated from each other to form separate ablation electrodes. An embodiment in which all elongated ablation electrode segments form independent electrodes, the planar member 100 includes a total of eight elongated ablation electrodes. The example ablation electrode configurations illustrated and described herein are non-limiting and numerous other ablation electrode configurations are possible. In each ablation electrode configuration, ablation electrodes can be paired following the same concepts as outlined in Table 1.

In addition to performing ablation between various electrodes on a single planar member 100, the disclosed technology can be further configured to perform ablation between electrodes on other planar members 100 of the expandable end effector portion 110. For example, as shown in FIGs. 12A and 12B, the expandable end effector portion 110 can include a plurality of planar members 100 (six planar members are shown in FIGs. 12A and 12B labeled as A-H, but it will be appreciated that the expandable end effector portion 110 can include more or less than six planar members 100) that each include electrodes according to any of the examples shown and described herein. The disclosed technology can be configured to perform ablation between electrodes on two or more of the planar members 100 of the expandable end effector portion 110. For example, as shown in FIG. 12A, electrodes on planar member 100 that are adjacent to each other can be electrically connected to perform ablation. For example, electrodes on planar member A can be electrically connected with electrodes on planar member B to perform ablation. The same can be accomplished with planar members C and D, planar members E and F, planar members G and H, planar members A and H, planar members G and F, planar members, E and D, and planar members C and B.

Alternatively, as shown in FIG. 12B ablation can be performed between electrodes on planar members 100 across the expandable end effector portion 110 from each other. For example, ablation can be performed between planar member A and planar member E, between planar member B and planar member F, between planar member C and planar member G, and between planar member D and planar member H.

As will be appreciated, the electrodes on planar members 100 can be configured to perform ablation on any of the other planar members. For example, electrodes on planar member A can be configured to perform ablation with electrodes on planar members, B, C, D, E, F, G, and/or H. Similarly, electrodes on planar member B can be configured to perform ablation with electrodes on planar members A, C, D, E, F, G, and/or H, and so on for the other planar members. That is, although various examples of performing ablation using electrodes on planar members AH are shown and described in FIGs. 12A and 12B, it will be appreciated that the disclosed technology can include performing ablation using any combination of the electrodes on planar members 100.

Furthermore, although shown as electrodes on only two planar members being configured to perform ablation together, it will be appreciated that various other combinations of electrodes on planar members 100 can be performed. For example, electrodes on one planar member (e.g., planar member A) can be configured to perform ablation with electrodes on two or more other planar members (e.g., planar members B and C; planar members B, C, and D, planar members B, C, D, and E, etc.). In other words, the electrodes on the various planar members 100 can be configured to perform ablation together to ablate a desired area of tissue and to achieve the desired ablation profile (lesion side, depth, etc.) for the particular application.

Further still, ablation can be completed by sequencing through various combinations of electrodes on planar members 100 as desired. For example, ablation can be completed using all of the electrodes on all of the planar members 100 by sequencing through various combinations of two planar members 100. For example, the sequence could include performing ablation between planar member A and planar member B, then between planar member C and planar member D, then between planar member E and planar member F, and then between planar member G and planar member H. Alternatively, the sequence could include performing ablation between planar member A and planar member C, then between planar member B and planar member D, then between planar member C and planar member E, then between planar member D and planar member F, then between planar member E and planar member G, then between planar member F and planar member H, then between planar member G and planar member A, and then between planar member H and planar member B. Alternatively, as yet another example, the sequence could include performing ablation between planar member A and planar member E, then between planar member B and planar member F, then between planar member C and planar member G, and then between planar member D and planar member H.

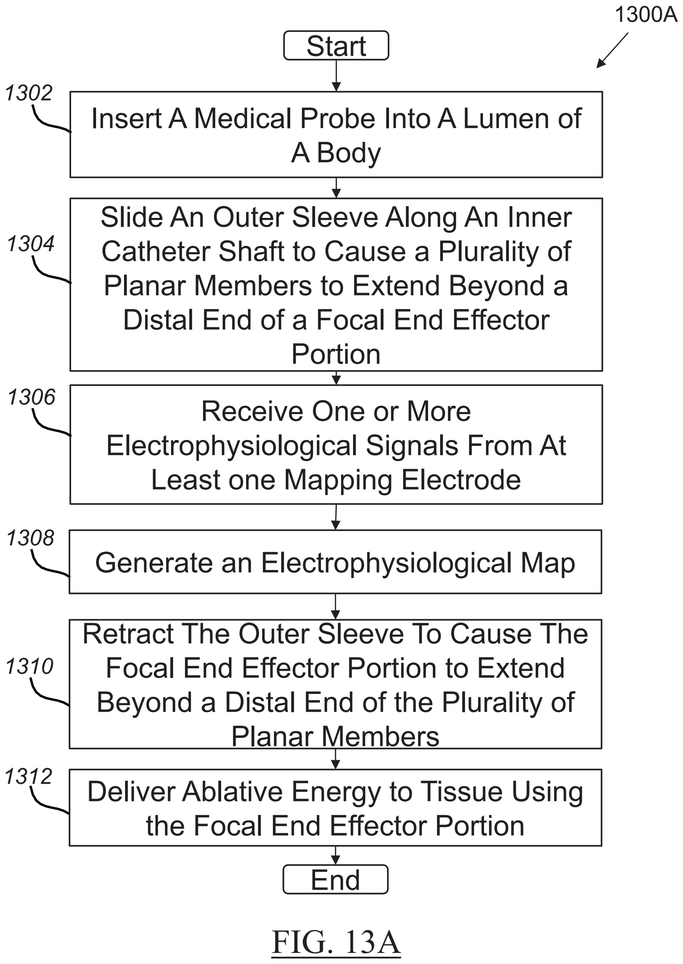

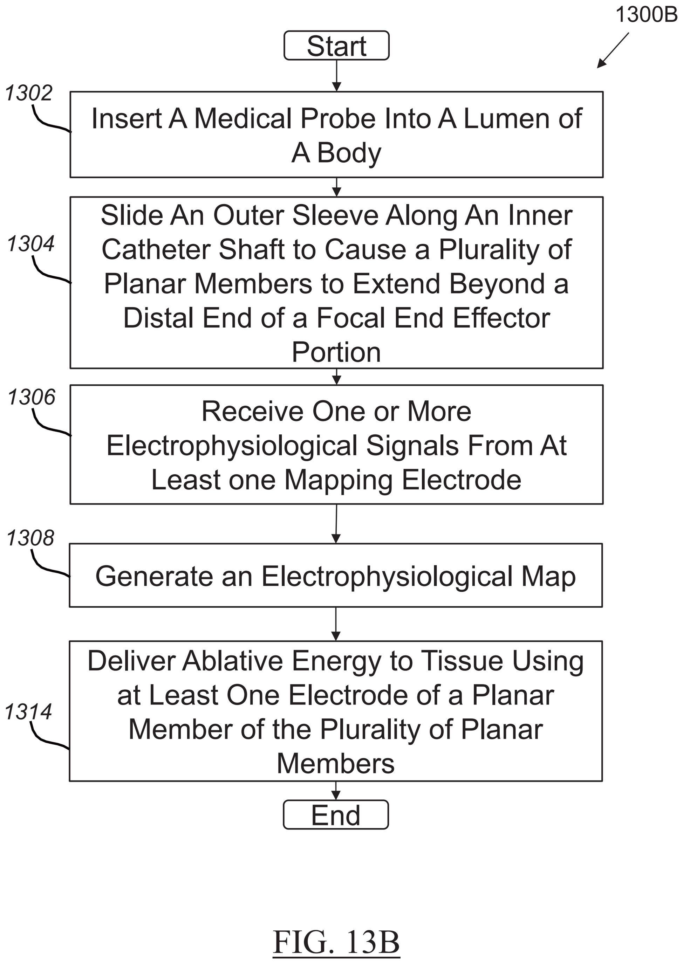

FIGs. 13A and 13B illustrate flow charts of methods 1300A and 1300B of performing mapping and ablation of tissue, in accordance with the disclosed technology. The methods 1300A and 1300B are offered for illustrative purposes and can include any of the disclosed features and methods previously described herein. Thus, the methods 1300A and 1300B should not be limited to the specific steps and order of steps shown and described herein.

The method 1300A shown in FIG. 13A can include inserting 1302 a medical probe into a lumen of a body, sliding 1304 an outer sleeve along an inner catheter shaft to cause an expandable end effector portion having plurality of planar members to extend beyond a distal end of a focal end effector portion. The method 1300A can further include receiving 1306 one or more electrophysiological signals from at least one mapping electrode disposed on the expandable end effector portion and generating 1308 a map representative of the electrophysiological signals propagating through tissue. The method 1300A can further include retracting 1310 the outer sleeve to cause the focal end effector portion to extend beyond a distal end of the plurality of planar members and delivering 1312 ablative energy to tissue via the focal end effector portion. As just described, the method 1300A can be used for completing a mapping procedure using an expandable end effector portion and then ablating tissue using a focal end effector portion to ablate relatively small areas of tissue. The ablation procedure can include ablating tissue at identified locations to stop the propagation of the aberrant signals through the tissue.

The method 1300B shown in FIG. 13B can include inserting 1302 a medical probe into a lumen of a body, sliding 1304 an outer sleeve along an inner catheter shaft to cause an expandable end effector portion having plurality of planar members to extend beyond a distal end of a focal end effector portion. The method 1300B can further include receiving 1306 one or more electrophysiological signals from at least one mapping electrode disposed on the expandable end effector portion and generating 1308 a map representative of the electrophysiological signals propagating through tissue. The method 1300B can further include delivering 1314 ablative energy to tissue using at least one electrode disposed a planar member of the plurality of planar members. As just described, the method 1300B can be used for completing a mapping procedure using an expandable end effector portion and then ablating tissue using the same expandable end effector portion to ablate relatively large areas of tissue. Ablating tissue with the method 1300B using the expandable end effector can include any of the various combinations of electrodes to perform ablation as previously described herein. The ablation procedure can include ablating tissue at identified locations to stop the propagation of the aberrant signals through the tissue.

As will be appreciated, the methods 1300A and 1300B just described are not intended to be limited to the particular steps described or to be limited to the particular order of steps described. That is, the methods 1300A and 1300B can include other intervening steps that are not explicitly described herein and/or can be executed in various orders. Accordingly, although the methods 1300A and 1300B are described as having particular steps and is presented in a particular an order, the methods 1300A and 1300B are not so limited.

The following clauses list non-limiting embodiments of the disclosure:
Clause 1: A medical probe comprising: an elongated shaft extending along a longitudinal axis; a distal electrode disposed at a distal end of the elongated shaft, the distal electrode configured to deliver ablative energy to tissue; an outer sleeve disposed at least partially around the elongated shaft; and a plurality of planar members attached to the outer sleeve and extending radially outward from the longitudinal axis, each planar member of the plurality of planar members comprising a framework coupled to an outer surface member and an inner surface member opposite the outer surface member, each of the inner and outer surface members includes a plurality of electrodes diametrically disposed on the inner surface member and outer surface member, the outer sleeve configured to move axially along the elongated shaft to move the plurality of planar members axially with respect to the distal electrode.
Clause 2: The medical probe of Clause 1, the outer sleeve configured to move the plurality of planar members beyond a distal end of the distal electrode.
Clause 3: The medical probe of any one of the preceding Clauses, at least one electrode of the plurality of electrodes on each respective planar member of the plurality of planar members being configured to detect electrophysiological signals.
Clause 4: The medical probe of any one of the preceding Clauses, at least one electrode of the plurality of electrodes on each respective planar member of the plurality of planar members being configured to deliver ablative energy to tissue.
Clause 5: The medical probe of Clause 4, wherein the ablative energy is configured to cause irreversible electroporation of the tissue.
Clause 6: The medical probe of any one of the preceding Clauses, the distal electrode being configured to deliver radio frequency (RF) ablative energy to tissue.
Clause 7: The medical probe of any of the preceding Clauses, wherein each planar member of the plurality of planar members comprises a first plurality of electrodes on a first side of the planar member and a second plurality of electrodes on a second side of the planar member.
Clause 8: The medical probe of Clause 7, wherein the first plurality of electrodes and the second plurality of electrodes are separated by a dielectric material.
Clause 9: The medical probe of any one the preceding Clauses, wherein each planar member of the plurality of planar members comprises a first flexible circuit on a first side of the planar member and a second flexible circuit on a second side of the planar member.
Clause 10: The medical probe of Clause 9, wherein the first flexible circuit and the second flexible circuit are separated by a dielectric material.
Clause 11: The medical probe of Clause 7 or Clause 10, wherein the dielectric material comprises a thermoplastic polyurethane.
Clause 12: The medical probe of Clause 11, wherein the dielectric material further comprises insulative material dispersed throughout at least a portion of the thermoplastic polyurethane.
Clause 13: The medical probe of any one of Clauses 7 to 12, further comprising one or more spines disposed in the dielectric material.
Clause 14: The medical probe of any one of the preceding Clauses, wherein each electrode of the plurality of electrodes comprises a serpentine shape.
Clause 15: The medical probe of any one of the preceding Clauses, wherein each electrode of the plurality of electrodes comprises a length at least twice as long as its width.
Clause 16: The medical probe of any one of the preceding Clauses, wherein each electrode of the plurality of electrodes comprises a conductive ink printed onto the respective planar member.
Clause 17: The medical probe of any one of the preceding Clauses, the plurality of electrodes on each respective planar member of the plurality of planar members being configured to deliver ablative energy to tissue, the medical probe further comprising: one or more processors; and memory storing instructions configured to, when executed by the one or more processors, control a delivery of the ablative energy to the plurality of electrodes.
Clause 18: The medical probe of Clause 17, wherein the instructions, when executed by the one or more processors, are configured to cause the medical probe to: deliver ablative energy between a first electrode and a second electrode of the planar member.
Clause 19: The medical probe of Clause 18, wherein the instructions, when executed by the one or more processors, are configured to cause the medical probe to: deliver ablative energy between a first electrode on a tissue-facing side of a given planar member and a second electrode on non-tissue-facing side of the given planar member.
Clause 20: The medical probe of Clause 18, wherein the first electrode and the second electrode are positioned on opposite sides of a plane extending vertically through the longitudinal axis of the planar member.
Clause 21: The medical probe of Clause 19, wherein the first electrode and the second electrode are positioned on the same side of a plane extending vertically through the longitudinal axis of the planar member.
Clause 22: A method comprising: inserting a medical probe into a lumen of a body, the medical probe comprising: an elongated shaft extending along a longitudinal axis; a distal electrode disposed at a distal end of the elongated shaft, the distal electrode configured to deliver ablative energy to tissue; an outer sleeve disposed at least partially around the elongated shaft; and a plurality of planar members attached to the outer sleeve and extending radially outward from the longitudinal axis, each planar member of the plurality of planar members comprising a plurality of electrodes, the outer sleeve configured to move axially along the elongated shaft to move the plurality of planar members axially with respect to the distal electrode; sliding the outer sleeve along the elongated shaft to cause the plurality of planar members to extend beyond a distal end of the distal electrode; and receiving one or more electrophysiological signals from at least one electrode of the plurality of electrodes disposed on the plurality of planar members.
Clause 23: The method of Clause 22 further comprising generating an electrophysiological map based at least in part on the one or more electrophysiological signals.
Clause 24: The method of Clause 22 or Clause 23 further comprising applying ablative energy to the tissue via at least one electrode of the plurality of electrodes.
Clause 25: The method of Clause 24, wherein the ablative energy is configured to cause irreversible electroporation of the tissue.
Clause 26: The method of Clause 24, wherein the ablative energy is conducted between a first electrode and a second electrode of the planar member.
Clause 27: The method of Clause 26, wherein the ablative energy is conducted between a first electrode on a tissue-facing side of a given planar member and a second electrode on non-tissue-facing side of the given planar member.
Clause 28: The method of Clause 27, wherein the first electrode and the second electrode are positioned on opposite sides of a plane extending vertically through the longitudinal axis of the planar member.
Clause 29: The method of Clause 27, wherein the first electrode and the second electrode are positioned on the same side of a plane extending vertically through the longitudinal axis of the planar member.
Clause 30: The method of Clause 22 or Clause 23 further comprising sliding the outer sleeve proximally along the elongated shaft to cause the distal electrode to extend beyond a distal end of the plurality of planar members.
Clause 31: The method of Clause 25 further comprising applying ablative energy to the tissue via the distal electrode.
Clause 32: The method of Clause 27, wherein the ablative energy comprises radio frequency (RF) ablative energy.

Having shown and described exemplary embodiments of the subject matter contained herein, further adaptations of the methods and systems described herein may be accomplished by appropriate modifications without departing from the scope of the claims. For instance, the planar member 100 may have an alternative shape or include alternative materials and the electrical signals used to achieve PFA may be modified to adapt to the specific geometry and materials of the planar member 100. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A medical probe comprising:
an elongated shaft extending along a longitudinal axis;
a distal electrode disposed at a distal end of the elongated shaft, the distal electrode configured to deliver ablative energy to tissue;
an outer sleeve disposed at least partially around the elongated shaft; and
a plurality of planar members attached to the outer sleeve and extending radially outward from the longitudinal axis, each planar member of the plurality of planar members comprising a framework coupled to an outer surface member and an inner surface member opposite the outer surface member, each of the inner and outer surface members includes a plurality of electrodes diametrically disposed on the inner surface member and outer surface member, the outer sleeve configured to move axially along the elongated shaft to move the plurality of planar members axially with respect to the distal electrode.

2. The medical probe of Claim 1, the outer sleeve configured to move the plurality of planar members beyond a distal end of the distal electrode.

3. The medical probe of Claim 1 or claim 2, at least one electrode of the plurality of electrodes on each respective planar member of the plurality of planar members being configured to detect electrophysiological signals.

4. The medical probe of any preceding Claim, at least one electrode of the plurality of electrodes on each respective planar member of the plurality of planar members being configured to deliver ablative energy to tissue, optionally wherein the ablative energy is configured to cause irreversible electroporation of the tissue.

5. The medical probe of Claim 1, wherein each planar member of the plurality of planar members comprises at least four electrodes on a first side of the planar member and at least four electrodes on a second side of the planar member.

6. The medical probe of Claim 1, wherein the plurality of electrodes on the inner surface member and the plurality of electrodes on the outer surface member are separated by a dielectric material.

7. The medical probe of any preceding Claim, wherein each planar member of the plurality of planar members comprises a first flexible circuit on a first side of the planar member and a second flexible circuit on a second side of the planar member.

8. The medical probe of Claim 7, wherein the first flexible circuit and the second flexible circuit are separated by a dielectric material.

9. The medical probe of Claim 8, wherein the dielectric material comprises a thermoplastic polyurethane.

10. The medical probe of Claim 9, wherein the dielectric material further comprises insulative material dispersed throughout at least a portion of the thermoplastic polyurethane.

11. The medical probe of any preceding Claim, wherein each electrode of the plurality of electrodes comprises a serpentine shape, a length at least twice as long as its width, or a conductive ink printed onto the respective planar member.

12. The medical probe of any preceding Claim, the plurality of electrodes on each respective planar member of the plurality of planar members being configured to deliver ablative energy to tissue, the medical probe further comprising:
one or more processors; and
memory storing instructions configured to, when executed by the one or more processors, control a delivery of the ablative energy to the plurality of electrodes to deliver ablative energy between a first electrode and a second electrode of the planar member.

13. The medical probe of Claim 12, wherein the instructions, when executed by the one or more processors, are configured to cause the medical probe to:
deliver ablative energy between a first electrode on a tissue-facing side of a given planar member and a second electrode on non-tissue-facing side of the given planar member.

14. The medical probe of Claim 12 or 13, wherein the first electrode and the second electrode are positioned on opposite sides of a center line of the planar member or on the same side of the centerline axis of the planar member.

15. The medical probe of Claim 1, the plurality of electrodes on each respective planar member of the plurality of planar members being configured to deliver ablative energy to tissue, the medical probe further comprising:
one or more processors; and
memory storing instructions configured to, when executed by the one or more processors, control a delivery of the ablative energy to the plurality of electrodes to deliver ablative energy between a first electrode disposed on a first planar member of the plurality of planar members and a second electrode disposed on a second planar member of the plurality of planar members.
